# EUROPÄISCHE PATENTSCHRIFT

(11) **EP 2 647 358 B1**
(45) Veröffentlichungstag und Bekanntmachung des Hinweises auf die Patenterteilung: **16.11.2016**
(21) Anmeldenummer: 13002316.1
(22) Anmeldetag: 02.06.2006
(51) Int. Cl.: A61F 9/08, A61N 1/36

(54) **Extraokulares Epiretinal-Implantat**
Extraocular epiretinal implant
Implant épirétinal extraoculaire

(30) Priorität: 14.07.2005 DE 102005032989
(43) Veröffentlichungstag der Anmeldung: 09.10.2013
(62) Teilanmeldung aus: 06754070.8
(73) Patentinhaber: Pixium Vision SA, 75012 Paris (FR)
(72) Erfinder: Zehnder, Thomas, CH-1206 Geneve (CH); Tiedtke, Hans-Jürgen, 53227 Bonn (DE)
(74) Vertreter: Graf von Stosch, Andreas

(56) Entgegenhaltungen:
- WO-A1-98/17343
- DE-A1- 10 315 397
- US-A1- 2002 091 421

## Beschreibung

Die vorliegende Erfindung betrifft eine Vorrichtung zur Implantation in einem menschlichen Auge mit einem Elektrodenarray bzw. einer Mikrokontaktstruktur zur Kontaktierung von Nervengewebe im visuellen System des menschlichen Auges. Die vorliegende Erfindung betrifft insbesondere eine Sehprothese mit einer Vorrichtung zum Erzeugen von Stimulations-Impulsen, die der Stimulation von lebendem Gewebe oder Nerven dienen.

Eine häufige Ursache für den teilweisen oder vollständigen Verlust des Sehvermögens ist die Zerstörung der Photorezeptorschicht in der Retina des menschlichen Auges, wonach auftreffende Photonen nicht in entsprechende Stimulation der Ganglienzellen umgesetzt werden. Bei diesem Krankheitsbild sind die Ganglienzellen nur teilweise betroffen, sodass eine externe Stimulation der in der Retina noch vorhandenen Ganglienzellen eine visuelle Wahrnehmung erzeugen kann. Auf dieser Basis werden seit einiger Zeit Entwicklungen vorgenommen, welche die Implantation einer Mikrokontaktstruktur zur Kontaktierung von intakten Ganglienzellen beinhalten.

Es wurden bereits Vorrichtungen in Form von Implantaten für die Netzhaut (Retina) des menschlichen Auges entwickelt, die zur Behandlung von Patienten vorgesehen sind, deren Sehvermögen teilweise oder vollständig durch Defekte in der Retina verloren gegangen ist. Dabei wird eine mikroelektronische Vorrichtung im Bereich der Retina mit einer Vielzahl von lichtempfindlichen Pixelelementen implantiert, über die ein auf die Retina durch die noch intakte Linse des Auges projiziertes Bild aufgenommen wird. Bei anderen Sehprothesen erfolgt die Bilderfassung durch eine externe Kamera, insbesondere eine Videokamera. Das durch die Pixelelemente bzw. die Kamera erfasste Bild wird in elektrische Signale umgesetzt und über Stimulations-Elektroden mittels elektrischer Stimulations-Impulse an die Ganglienzellen der Retina bzw. an den Sehnerv abgegeben, um so das Sehvermögen des erblindeten oder teilweise erblindeten Patienten wiederherzustellen bzw. zu verbessern.

Zur epiretinalen Übertragung der Stimulations-Impulse an die Zellen der Retina bzw. an die Zellen der Sehnerven werden Mikrokontaktstrukturen verwendet, die im Wesentlichen aus einem Trägermaterial bestehen, das einseitig elektrisch leitende, stift- oder nadelförmig ausgebildete Kontaktelemente trägt, die über die Ebene der Trägerfolie hervorstehen und gleichmäßig mit einer konstanten Flächendichte über die Oberfläche des Implantats verteilt sind. Die bekannten Sehprothesen haben jedoch den Nachteil, dass sie mit einem hohen Platzbedarf verbunden sind. Aufgrund der besonderen Sensibilität des menschlichen Auges und dem äußerst begrenzten Raum im Augeninneren ist es grundsätzlich erstrebenswert, Stimulations-Systeme bzw. die Implantate der Sehprothesen auf möglichst kleinem Raum unterzubringen.

Ein weiteres Problem bekannter Sehprothesen besteht darin, die Implantate und deren Mikrokontaktstruktur bzw. die Fläche der Elektroden mit Energie zu versorgen. Nach gegenwärtigem Kenntnisstand ist für die Energieversorgung eines Retina-Implantats eine mittlere Leistung von ca. 40 mW erforderlich. Eine solche Energieversorgung kann über einen längeren Zeitraum nicht über eine implantierte Batterie bewerkstelligt werden, da diese einen zu großen Platzbedarf benötigen würde.

Für aktive Retina-Implantate ist daher eine vom System zur Generierung des Seheindrucks unabhängige Energieversorgungseinheit erforderlich, die sich außerhalb des Auges befindet und ohne Drahtverbindung zum Retina-Implantat arbeitet. Aus der DE 19705988 C2 ist ein subretinales Implantat bekannt, wobei das Implantat mit einer für nicht sichtbares Licht wirksamen, photovoltaischen Schicht versehen ist. Hierbei wird die Energieversorgung über Infrarotlicht vorgenommen. Das Retina-Implantat ist mit einer an einer Netzhaut anliegenden Oberfläche versehen, wobei die Oberfläche mit Elektroden zum Stimulieren von Zellen der Netzhaut versehen ist. Die Stromversorgung der Komponenten des Implantats im Inneren des Auges über Infrarotlicht kann jedoch die Gefahr von thermischen Schädigungen des Auges durch lokale Erwärmungen im Augeninneren mit sich bringen.

Die Druckschrift US 2002/091421 A beschreibt eine Sehprothese mit einem Stimulations-System zur Implantation in einem menschlichen Auge, mit einem Elektrodenarray zur Kontaktierung und Stimulation von lebendem Gewebe oder Nerven im visuellen System des Auges, das mittels einer elektrischen Steuereinheit Stimulations-Impulse erzeugt, wobei das Stimulations-System ein intraokulares Implantat und ein extraokulares Implantat umfasst, welches das intraokulare Implantat mit Energie versorgt.

Die Patentanmeldung DE 103 15 397 A1 beschreibt eine Energieversorgungsvorrichtung für ein Retina-Implantat, bei welcher eine Infrarot-Lichtquelle zur Energieversorgung des Retina-Implantats vorgesehen ist. Die Lichtquelle wird vor dem Auge, insbesondere in einer Brille angeordnet.

Eine Aufgabe der vorliegenden Erfindung besteht folglich darin, eine Sehprothese in Form eines Retina-Implantats bereitzustellen, das sich durch möglichst geringen Platzbedarf im Inneren des Auges auszeichnet. Eine weitere Aufgabe der vorliegenden Erfindung besteht darin, ein Implantat-System bereitzustellen, dessen Stromversorgung die Bewegungsfreiheit des Auges in der Augenhöhle möglichst wenig behindert.

Diese Aufgabe wird nach der vorliegenden Erfindung durch eine Sehprotese mit den Merkmalen gemäß Anspruch 1 gelöst. Vorteilhafte Weiterbildungen der Erfindung sind in den Unteransprüchen angegeben. Die vorliegende Erfindung löst die oben genannte Aufgabe durch eine Sehprothese mit einem Stimulations-System zur Implantation in einem menschlichen Auge, mit einem Elektrodenarray zur Kontaktierung und Stimulation von lebendem Gewebe oder Nerven im visuellen System des Auges, das mittels einer elektrischen Steuereinheit Stimulations-Impulse erzeugt, wobei das Stimulations-System zumindest ein intraokulares Implantat und zumindest ein extraokulares Implantat umfasst, welches das intraokulare Implantat mit Energie versorgt, wobei der Licht-Empfänger des intraokularen Implantats als Infrarot-Empfänger ausgebildet ist, Infrarot-Signale eines Infrarot-Senders von außerhalb des Auges, vorzugsweise über den natürlichen Lichtweg des Auges zu empfangen.

Die vorliegende Erfindung stellt ein Neurostimulations-Gerät zur Stimulation noch vorhandener Ganglien-Nervenzellen bereit, das die Sehfähigkeit bei degenerativen Netzhautleiden, aber noch intakten Sehnerven verbessern kann. Durch das Aufteilen des Implantats in einen epiretinalen Teil und einen extraokularen Teil kann eine Vielzahl der erforderlichen Komponenten und das größte Volumen des Implantats auf den äußeren, den extraokularen Teil des Implantats verlagert werden. Mit Hilfe des erfindungsgemäßen Implantats können potentielle Verletzungen der Retina oder anderer sensibler Strukturen des Auges bei der Anordnung des Stimulations-Systems minimiert werden.

Die erfindungsgemäße Sehprothese bietet somit den Vorteil, dass nahezu sämtliche Elektronik-Bauteile, die nicht zwingend mit dem intraokularen Implantat im Augeninneren untergebracht werden müssen, außerhalb des Augapfels, beispielsweise an der sogenannten Sklera bzw. Lederhaut angeordnet werden können. Auf diese Weise wird der Platzbedarf des Stimulations-Systems im Augeninneren verringert und der operative Eingriff beim Implantieren des Stimulations-Systems im Inneren des Auges möglichst klein gehalten. Ein weiterer Vorteil der erfindungsgemäßen Sehprothese ist darin zu sehen, dass die Stromversorgung des intraokularen Implantats über das extraokulare Implantat erfolgen kann, ohne die Bewegungsfreiheit des Auges in der Augenhöhle zu behindern. Die erfindungsgemäße Sehprothese erlaubt außerdem eine weitgehend verletzungsfreie Wartung oder Austausch des Stimulations-Systems, wenn beispielsweise das extraokulare Implantat durch eine modernere Version ersetzt werden soll.

Der extraokulare Teil des Implantats ist so am äußeren Umfang des Auges an der Lederhaut (Sklera) angeordnet, dass die Bewegung des Augapfels möglichst wenig beeinträchtigt wird. Besonders vorteilhaft ist es, wenn der extraokulare Teil des Implantats in dem das Auge umgebende Fettgewebe zwischen zwei Muskeln platziert wird, die zur Bewegung des Auges dienen. Dabei kann das extraokulare Implantat von außen an die Lederhaut des Auges angenäht werden. Auf diese Weise ist eine behinderungs- und schmerzfreie Bewegung des Auges innerhalb der Augenhöhle möglich.

Die einzelnen Implantatteile innerhalb und außerhalb des Auges sind vorzugsweise über eine Drahtverbindung (mit oder ohne Stecker) miteinander koppelbar. Im implantierten Zustand des erfindungsgemäßen Implantats wird diese Drahtverbindung vorzugsweise im Bereich der Parsplana durch das Auge geführt, in der Nähe der Regenbogenhaut, wo keine Netzhaut vorhanden ist. Sowohl die Übertragung der Energie bzw. der Stromversorgung als auch der Bild-Daten zwischen dem extraokularen Implantat außerhalb des Auges und der weiteren Elektronik kann drahtlos auf induktivem Wege stattfinden. Die drahtlose Übertragung von Energie und Bild-Daten von der vom Auge entfernten Elektronik zu dem Implantat vermeidet Kabelbewegungen und damit einhergehende Behinderungen oder Verletzungen.

Die weitere Elektronik der Sehprothese, die zur Bearbeitung und Aufbereitung der von einer externen Kamera aufgenommenen Bild-Daten erforderlich ist, kann vom Auge entfernt außerhalb des Körpers angeordnet werden. Die elektronischen Komponenten können beispielsweise in einem sogenannten Pocket-Computer untergebracht werden, der in einer separaten Tasche am Körper transportiert werden kann. Besonders vorteilhaft sind die elektronischen Komponenten in einem Brillengestell untergebracht, in dem sich auch die Kamera befindet, welche die Bild-Daten erfasst.

Da sich die elektronischen Komponenten, die zur Bildverarbeitung der von der Videokamera gelieferten Signale benötigt werden, außerhalb des Körpers befinden, können diese leicht gewartet werden bzw. durch eine modernere Version der elektronischen Schnittstelle ersetzt werden. Die Komponenten der elektronischen Schnittstelle können individuell auf den jeweiligen elektronischen Stimulations-Pegel des Implantat-Systems angepasst werden. Dadurch kann ein minimaler Pegel elektrischer Ladung für sämtliche Elektroden in dem Elektrodenarray gewährleistet werden, um das durch die elektrischen Stimulations-Impulse stimulierte Gewebe bzw. Nervenzellen möglichst wenig zu belasten. Auf diese Weise werden durch einen überhöhten Ladungs-Pegel verursachte Schäden auf der Netzhaut des Auges in der Nähe der Elektroden sowie Schmerzempfindungen des Patienten vermieden.

Bei dem erfindungsgemäßen Stimulations-System wird im Prinzip mit einer externen Kamera eine Bildaufnahme vorgenommen, deren Bild-Signale nach einer elektronischen Vorverarbeitung über das extraokulare Implantat und das epiretinale Implantat der Netzhaut des Auges zugeführt werden. Das epiretinale Implantat umfasst ein integriertes Elektrodenarray, das entsprechend den empfangenen Bild-Daten ortsaufgelöst die Ganglienzellen bzw. die Zellen der Retina durch elektrische Signale reizt und damit das von der externen Kamera aufgenommene Bild an die Nerven des visuellen Systems weitergibt. Ein besonderer Vorteil des aktiven epiretinalen Implantats besteht darin, dass es sich an verschiedene Bedingungen hinsichtlich der Umgebungsleuchtdichte anpassen bzw. adaptieren kann.

Das intraokulare Implantat umfasst ein Elektrodenarray mit einer Anzahl von Stimulations-Elektroden die vorzugsweise in einer Matrix dicht nebeneinander angeordnet sind. Das Elektrodenarray umfasst eine Mikrokontaktstruktur eine mit einer Anzahl von Kontaktstellen, über die die Stimulations-Elektroden mit Retinazellen bzw. Ganglienzellen in Kontakt stehen und die kontaktierten Retinazellen bzw. Ganglienzellen mittels Stimulations-Impulsen stimulieren. Der Außenbereich der Mikrokontaktstruktur zur epiretinalen Kontaktierung der Ganglienzellen ist der Außenkontur des fovealen Bereichs der Retina angepasst und kann sphärische Form haben. Dabei ist die Mikrokontaktstruktur bzw. das Elektrodenarray des epiretinalen Implantats vorzugsweise im Bereich der Makula des Auges angeordnet. Die Makula ist die Stelle im Augeninneren bzw. auf der Netzhaut, auf die das meiste Licht fällt; sie wird deshalb auch als die "Stelle des schärfsten Sehens" bezeichnet.

Das extraokulare Implantat ist mit einer elektrischen Steuereinheit ausgestattet, die vorzugsweise als digitale Steuereinheit mit analogen Zusatzfunktionen ausgebildet ist und Stimulations-Impulse anhand der von einer externen Kamera aufgenommenen Bild-Daten erzeugt. Dazu umfasst die elektrische Steuereinheit mindestens eine Strom- bzw. Spannungsquelle und mindestens einen Impulsgenerator, der elektrische Stimulations-Impulse erzeugt, die von der Strom/Spannungsquelle zu Stimulations-Impulsen bzw. Stimulations-Strömen verstärkt und an die Stimulations-Elektroden in dem Elektrodenarray im intraokularen Implantat weitergeleitet werden. Zusätzlich kann die elektrische Steuereinheit mit elektronischen Speichermitteln ausgestattet sein, in denen die berechnete Dauer und Intensität der zu erzeugenden Stimulations-Impulse gespeichert werden und auf einen bestimmten Befehl hin abgerufen werden können. Die elektronischen Komponenten der elektrischen Steuereinheit sind zweckmäßigerweise zumindest teilweise in einem integrierten Schaltkreis photolithographisch mikrostrukturiert und vorzugsweise auf einem Chip in dem extraokularen Implantat untergebracht. Das extraokulare Implantat besitzt mindestens eine Gegenelektrode, die als Rückstrompfad für die Stimulationselektroden dient.

Die elektrische Steuereinheit hat für jede Stimulations-Elektrode ein Kontaktpad, d.h. eine Anschlussfläche, über die jeweils eine Stimulations-Elektrode mittels einer separaten Drahtverbindung kontaktiert werden kann. Die Drahtverbindung ist als flexibles Implantat ausgebildet und wird zwischen dem extraokularen Implantat und dem intraokularen Implantat vorzugsweise im Bereich der Parsplana in das Augeninnere geführt, wo keine Netzhaut vorhanden ist, so dass eine Beeinträchtigung der Retina vermieden wird.

Die Führung der Drahtverbindung zwischen dem epiretinalen Implantat und dem extraokularen Implantat durch die Lederhaut (Sklera) des Auges im Bereich der Parsplana stellt einen Eingriff mit dem geringsten Aufwand und der geringst möglichen Verletzung des Auges dar. Dadurch wird auch die Gefahr von Komplikationen und das Infektionsrisiko bei der Operation verringert. Indem das flexible Implantat der Drahtverbindung zusammen mit dem inneren und dem äußeren Teil des Implantats am Auge befestigt sind, vollziehen diese die gleichen Bewegungen des Auges, wodurch die Bewegungsfreiheit des Auges weder durch die Drahtverbindung noch durch den inneren und äußeren Teil des Implantats beeinträchtigt wird.

Die Drahtverbindung zur Kopplung des extraokularen Implantats mit dem intraokularen Implantat umfasst mindestens eine Leitung zur Übertragung des Betriebsstroms und mindestens eine Signalleitung zur Übertragung von Bild-Daten und/oder elektrischer Stimulations-Impulse von der digitalen Steuereinheit an das intraokulare Implantat. Gemäß einer bevorzugten Ausführungsform der vorliegenden Erfindung umfasst die Drahtverbindung neben den elektrischen Leitungen zur Übertragung des Betriebsstrom mindestens so viele Leitungen zur Übertragung elektrischer Stimulations-Impulse, wie Stimulations-Elektroden in dem intraokularen Implantat vorgesehen sind. Ferner kann die Drahtverbindung einen oder mehrere Lichtleiter zur eindirektionalen oder bidirektionalen Datenübertragung mittels Lichtsignalen zwischen dem extraokularen Teil und dem intraokularen Teil des Implantats umfassen.

Um eine zuverlässige Fixierung des flexiblen Implantats mit dem Elektrodenarray bzw. der Mikrokontaktstruktur und der Drahtverbindung zwischen der Mikrokontaktstruktur und dem extraokularen Implantat zu gewährleisten, kann das intraokulare Implantat und/oder das flexible Implantat der Drahtverbindung mit Hilfe eines Nagels, einem sogenannten Tack, am Augeninneren fixiert werden. Dazu wird der Nagel operativ aus dem Augeninneren gesetzt und verläuft durch das flexible Implantat und die Netzhaut bis in die Aderhaut oder die Lederhaut des Auges, wo er sich mit seinen Widerhaken verankert.

Das intraokulare Implantat umfasst eine Anzahl von lichtempfindlichen Elementen, die in Abhängigkeit von auf dem intraokularen Implantat auftreffenden Licht die Kontaktstellen des Elektrodenarrays über die elektrische Schaltung ansteuern. Dabei ist mindestens ein Licht-Empfänger des intraokularen Implantats in der Lage, Licht-Signale eines Licht-Senders von außerhalb des Auges zu empfangen. Erfindungsgemäß ist der Licht-Empfänger des intraokularen Implantats als Infrarot-Empfänger ausgebildet, der Infrarot-Signale eines Infrarot-Senders von außerhalb des Auges, vorzugsweise über den natürlichen Lichtweg des Auges empfängt.

Auf diese Weise kann die Schnittstelle zwischen dem Licht-Sender außerhalb des Auges und den lichtempfindlichen Elementen bzw. dem Licht-Empfänger des intraokularen Implantats von einer externen Kamera aufgenommene Bild-Daten über Licht-Signale vom Licht-Sender außerhalb des Auges an die lichtempfindlichen Elemente bzw. den Licht-Empfänger des intraokularen Implantats übermitteln. Zur Übertragung der Bild-Daten wird vorzugsweise Infrarot-Licht verwendet, da dieses außerhalb des sichtbaren Lichtspektrums liegt und somit eine verbliebene Sehfähigkeit des Patienten und die Übertragung der Bild-Daten nicht irritiert.

In dem extraokularen Implantat erfolgt eine Signalbearbeitung der empfangenen Bild-Daten einschließlich einer Signalverstärkung, weshalb eine externe Energieeinkopplung erforderlich wird. Diese Energieeinkopplung erfolgt bei der erfindungsgemäßen Sehprothese drahtlos durch die induktive Schnittstelle zwischen einer externen Hochfrequenz-Sende-Spule und der Hochfrequenz-Empfänger-Spule des extraokularen Implantats. Dazu ist gemäß einer weiteren bevorzugten Ausführungsform der erfindungsgemäßen Sehprothese eine vom Stimulations-System entfernte Antenne für eine induktive Schnittstelle vorgesehen, die elektromagnetische Signale vorzugsweise im Hochfrequenz-Bereich senden kann. Ferner ist das extraokulare Implantat mit einer Antenne ausgestattet, die elektromagnetische Signale vorzugsweise im Hochfrequenz-Bereich empfangen kann.

Die Hochfrequenz-Antenne des extraokularen Implantats empfängt die von der Sende-Antenne der Elektronik außerhalb des Körpers ausgestrahlten elektromagnetischen Hochfrequenz-Signale. Dadurch entsteht ein induktiver Strom, der das Implantat am Auge mit ausreichender Energie versorgt. Der durch die Induktion entstehende Strom wird vom äußeren Teil des Implantats über die Drahtleitung an den inneren Teil des Implantats übermittelt, um das Elektrodenarray und den Infrarot-Empfänger mit Strom zu versorgen.

Zusätzlich kann die induktive Schnittstelle zwischen der Antenne außerhalb des Auges und der Antenne des extraokularen Implantats auch bidirektional ausgebildet sein, indem die vom Stimulations-System entfernte Antenne elektromagnetische Signale vorzugsweise im Hochfrequenz-Bereich empfangen kann und die Antenne des extraokularen Implantats elektromagnetische Signale vorzugsweise im Hochfrequenz-Bereich senden kann. Bei dieser bevorzugten Ausführungsform ist das extraokulare Implantat so ausgebildet, dass es über die induktive Schnittstelle Informationen, beispielsweise zu Betriebsparametern des Stimulations-Systems übermitteln kann. Gemäß einer weiteren besonderen Ausführungsform der Erfindung ist die Datenrate der von der Antenne des extraokularen Implantats empfangenen Signale von der Datenrate der von der Antenne des extraokularen Implantats gesendeten Signale unterschiedlich.

Bei einer bidirektionalen, induktiven Schnittstelle ist folglich sowohl der äußere Teil des Implantats am Auge als auch die Elektronik außerhalb des Körpers mit einer Sende- und einer Empfangseinheit ausgestattet, die jeweils elektrische Signale vorzugsweise im Hochfrequenzbereich senden und empfangen kann. Dadurch können auch von dem epiretinalen Implantat im Inneren des Auges erzeugte Signale über die Drahtleitung an den äußeren Teil des Implantats übermittelt und von dort über die Sendeeinheit in Form von Hochfrequenz-Signalen an die Elektronik außerhalb des Körpers weitergeleitet werden.

Die Elektronik außerhalb des Körpers empfängt die Hochfrequenz-Signale vom Sender des extraokularen Implantats über ihre Empfängereinheit und leitet diese einer zentralen Recheneinheit zu, wo die Signale ausgewertet werden. Auf diese Weise können vom epiretinalen Implantat im Inneren des Auges Signale übermittelt werden, die z.B. über eine ausreichende Stromversorgung des internen Implantats, die Qualität der empfangenen Bildsignale, die Funktion der Stimulations-Elektroden im Elektrodenarray, die Effizienz der induktiven Schnittstelle oder den Kontakt der Stimulations-Elektroden zu den Ganglienzellen Auskunft geben.

Das intraokulare Implantat umfasst mindestens ein lichtemittierendes Element, das Licht-Signale in Abhängigkeit von Betriebsparametern des Stimulations-Systems ausstrahlt. Dazu sind die von dem lichtemittierenden Element abgestrahlten Licht-Signale in Abhängigkeit von Betriebsparametern des intraokularen Implantats beispielsweise durch Modulation der Dauer und/oder der Intensität der Licht-Signale codiert. Die von dem lichtemittierenden Element abgestrahlten Licht-Signale können beispielsweise Informationen über die Position des intraokularen Implantats, über die Qualität der von dem intraokularen Implantat empfangenen Bild-Daten, über die Qualität der Stromversorgung des intraokularen Implantats und/oder über die Impedanz bzw. den elektrischen Widerstand der Stimulations-Elektroden enthalten. Die von dem lichtemittierenden Element abgestrahlten Licht-Signale können ferner Informationen über die Funktion der Stimulationselektroden im Elektrodenarray sowie über den Kontakt der Stimulationselektroden zu den Ganglienzellen enthalten.

Dieses lichtemittierende Element ist so im Augeninneren angeordnet, dass die von dem lichtemittierenden Element abgestrahlten Licht-Signale von einem Beobachter über Sichtkontakt in das Augeninnere detektierbar sind. Das lichtemittierende Element ist vorteilhaft als Diode (Status-Diode) ausgebildet ist, die Licht, insbesondere Infrarotlicht ausstrahlt, das von einem Licht-Empfänger, insbesondere von einem Infrarotlicht-Empfänger außerhalb des Auges detektierbar ist.

Nach einem weiteren Aspekt der vorliegenden Erfindung werden die oben genannten Aufgaben ferner gelöst durch ein Verfahren zum Betreiben der erfindungsgemäßen Vorrichtung umfassend zumindest die folgenden Schritte:
- Erfassen eines Bildes durch eine externe Kamera,
- Erzeugen von ortsaufgelösten Bild-Daten aus dem erfassten Bild,
- Berechnen von Diagnosebefehlen, Steuerbefehlen oder Stimulationsbefehlen bestimmter Dauer und Intensität in Abhängigkeit von den Bild-Daten,
- Übermitteln der Diagnosebefehle, Steuerbefehle oder Stimulationsbefehle an ein Stimulations-System mit einem intraokularen Implantat und einem extraokularen Implantat,
- Berechnen und Erzeugen elektrischer Stimulations-Impulse bzw. Stimulations-Ströme bestimmter Dauer und Intensität in dem extraokularen Implantat oder Ausführen von Diagnoseaufgaben entsprechend den Diagnosebefehlen, Steuerbefehlen oder Stimulationsbefehlen,
- Übermitteln der elektrischen Stimulations-Impulse bzw. Stimulations-Ströme an das intraokulare Implantat, und
- Anlegen der elektrischen Stimulations-Impulse bzw. Stimulations-Ströme an mindestens eine Stimulations-Elektrode im intraokularen Implantat, wodurch zumindest eine Retinazelle bzw. Ganglienzelle stimuliert wird, die mit der betreffenden Stimulations-Elektrode in Kontakt steht.

Um die von der externen Kamera aufgenommenen Bild-Daten auf die Verwendung in dem Stimulations-System vorzubereiten, werden diese in der elektrischen Steuereinheit vor der Übermittlung an das Stimulations-System elektrisch ausgewertet bzw. bearbeitet, um entsprechende elektrische Stimulations-Impulse bzw. Stimulations-Ströme zu erzeugen. Dabei können die Komponenten der elektrischen Steuereinheit einerseits Teil des extraokularen Implantats sein oder andererseits in einer externen Rechnereinheit untergebracht sein, die separat vom Patienten mitgeführt wird oder in einer Brille untergebracht sein, an der auch die externe Kamera und/oder der Licht-Sender für die Infrarot-Schnittstelle angeordnet ist.

Wie oben beschrieben, erfolgt bei dem Verfahren zum Betreiben der erfindungsgemäßen Vorrichtung die Übertragung des zum Betrieb des extraokularen Implantats und des intraokularen Implantats erforderliche Stroms drahtlos über eine induktive Schnittstelle zwischen der Hochfrequenz-Sender-Antenne außerhalb des Auges und der Hochfrequenz-Empfänger-Antenne des extraokularen Implantats, während die Übertragung der von der externen Kamera aufgenommenen Bild-Daten drahtlos über eine Infrarot-Schnittstelle zwischen dem Infrarot-Sender außerhalb des Auges und dem Infrarot-Empfänger innerhalb des Auges erfolgt. Alternativ kann die Übertragung der von der externen Kamera aufgenommenen Bild-Daten ebenfalls drahtlos über die induktive Schnittstelle zwischen der Hochfrequenz-Sender-Antenne außerhalb des Auges und der Hochfrequenz-Empfänger-Antenne des extraokularen Implantats erfolgen.

Die Übertragung der von der externen Kamera aufgenommenen Bild-Daten bzw. der Diagnosebefehle, Steuerbefehle oder Stimulationsbefehle kann als serieller Datenstrom vom Infrarot-Empfänger innerhalb des Auges über die Drahtverbindung an die digitale Steuereinheit im extraokularen Implantat erfolgen. Dabei enthält der serielle Datenstrom vom Infrarot-Empfänger innerhalb des Auges über die Drahtverbindung an die digitale Steuereinheit im extraokularen Implantat Informationen über die Elektrodenadresse, z.B. 1 bis 250, und über die zu der Elektrodenadresse dazugehörige Amplitude, z.B. 0 bis 1000µA, der Stimulations-Impulse für die betreffende Stimulations-Elektrode. Anhand der Informationen bezüglich der Elektrodenadresse und der Amplitude der Stimulations-Impulse werden von der elektrischen Steuereinheit des extraokularen Implantats für jede Stimulations-Elektrode Stimulations-Impulse bestimmter Dauer und Intensität berechnet und erzeugt. Die Form bzw. der Verlauf der elektrischen Stimulations-Impulse ist an die zu stimulierenden Ganglienzellen angepasst. Über eine Vielzahl von Stromgeneratoren in dem extraokularen Implantat werden die Stimulations-Elektroden mit elektrischem Strom bestimmter Intensität und Dauer beaufschlagt.

Die Stimulations-Impulse bzw. Stimulations-Ströme werden von der elektrischen Steuereinheit des extraokularen Implantats als paralleler Signalstrom über parallele Drahtverbindungen an die Stimulations-Elektroden im intraokularen Implantat übermittelt. Zu diesem Zweck verfügt die elektrische Steuereinheit des extraokularen Implantats bzw. der Retina-Stimulator-Chip beispielsweise über 250 Anschlusspads, an die jeweils Drähte für 250 Stimulations-Elektroden im Elektrodenarray des intraokularen Implantats anschließbar sind.

Bei einer bevorzugten Ausführungsform des Verfahrens ist es auch möglich, dass das intraokulare Implantat Diagnose-Daten bezüglich der Betriebsparameter des intraokularen Implantats über die Drahtverbindung an das extraokulare Implantat, beispielsweise als serieller Datenstrom übermittelt. Anschließend wird der serielle Diagnose-Datenstrom von der extraokularen Induktionsspule induktiv, z.B. durch Lastmodulation, an externe Diagnose-Mittel weitergeleitet, die beispielsweise in der Brille untergebracht sind. Alternativ zu dem oben beschriebenen induktiven Rückkopplungsweg kann auch die Status-Leuchtdiode im intraokularen Teil des Implantats als optischer Rückkanal mit digitaler Empfangseinheit und digitaler Auswerteeinheit in der Brille verwendet werden.

### Weitere bevorzugte Ausführungsformen des erfindungsgemäßen Implantats

Wie oben bereits beschrieben, können insbesondere Elektronik-Komponenten der erfindungsgemäßen Sehprothese in einer Baugruppe außerhalb des Körpers, vorzugsweise in einer Brille untergebracht sein, die der Patient wie eine normale Sehhilfe tragen kann. Nachfolgend werden die in einer Baugruppe außerhalb des Körpers untergebrachten Elektronik-Komponenten als extrakorporaler Teil der erfindungsgemäßen Sehprothese bezeichnet, während die innerhalb des Körpers angeordneten Komponenten der erfindungsgemäßen Sehprothese, umfassend die intraokular im Auge und die extraokular am Augapfel implantierten Komponenten als intrakorporaler Teil der erfindungsgemäßen Sehprothese zusammengefasst werden.

Zwischen dem extrakorporalen Teil (bspw. in der Brille) der Sehprothese und dem intrakorporalen Teil im bzw. am Auge des Patienten besteht eine drahtlose induktive Schnittstelle, über die sowohl eine Energieeinkopplung als auch eine Datenübertragung stattfindet. Gemäß einer bereits beschriebenen bevorzugten Ausführungsform der vorliegenden Erfindung ist diese induktive Schnittstelle zwischen dem extrakorporalen Teil und dem intrakorporalen Teil bidirektional ausgebildet. Dazu ist sowohl der extrakorporale Teil als auch der intrakorporale Teil mit einer Sende-Spule ausgestattet, die elektrische Signale vorzugsweise im Hochfrequenzbereich senden kann, und mit einer Empfänger-Spule bzw. einer Antenne ausgestattet, die elektrische Signale vorzugsweise im Hochfrequenz-Bereich senden kann. Auf diese Weise können elektrische Signale sowohl vom extrakorporalen Teil an den intrakorporalen Teil als auch in umgekehrter Richtung vom intrakorporalen Teil an den extrakorporalen Teil der erfindungsgemäßen Sehprothese übermittelt werden (Bidirektionalität). Sowohl beim extrakorporalen Teil als auch beim intrakorporalen Teil kann auch nur eine Sende-Empfänger-Spule vorgesehen sein, die jeweils beide Funktionen des Sendens und Empfangens von elektrischen Signalen erfüllt.

In einer Weiterbildung dieser bevorzugten Ausführungsform der vorliegenden Erfindung umfasst die bidirektionale Datenleitung zwischen dem extrakorporalen Teil und dem intrakorporalen Teil der Sehprothese mindestens zwei, vorzugsweise drahtlose Übertragungskanäle. Dabei verläuft mindestens ein drahtloser Übertragungskanal vom dem extrakorporalen Teil (bspw. in der Brille) zu dem intrakorporalen Teil der Sehprothese im Auge, nachfolgend auch als Hinübertragungskanal (Up-Link) bezeichnet, und mindestens ein drahtloser Übertragungskanal vom intrakorporalen Teil der Sehprothese im Auge zu dem extrakorporalen Teil in der Brille, nachfolgend auch als Rückübertragungskanal (Down-Link) bezeichnet.

Die Datenübertragung zwischen dem extrakorporalen Teil und dem intrakorporalen Teil der Sehprothese erfolgt vorzugsweise simultan, d.h. sowohl auf dem der Hinübertragungskanal als auch über den Rückübertragungskanal werden Daten gleichzeitig übertragen. Der Rückübertragungskanal kann insbesondere benutzt werden, um Daten über den Status des intrakorporalen Teils der Sehprothese zu übermitteln. Auf diese Weise wird ein zusätzlicher Sicherheitsgewinn erzielt, indem der Zustand und die Funktionalität des intrakorporalen Teils der Sehprothese ständig überwacht und bei einem Ausfall der Sehprothese oder des Rückübertragungskanal eine entsprechende Fehlfunktion signalisiert werden kann.

Bei einer weiteren bevorzugten Ausführungsform der vorliegenden Erfindung kann die Datenübertragung zwischen dem extrakorporalen Teil und dem intrakorporalen Teil der Sehprothese abwechselnd erfolgen. So kann beispielsweise während bestimmter Zeiträume der Hinübertragungskanal vom extrakorporalen Teil zum intrakorporalen Teil der Sehprothese aktiv sein und während anderer bestimmter Zeiträume der Rückübertragungskanal vom intrakorporalen Teil zum extrakorporalen Teil der Sehprothese aktiv sein. Bei dieser alterierenden Datenübertragung kann auch nur ein Übertragungskanal vorgesehen sein, da dieser dann alternativ entweder als Hinübertragungskanal oder als Rückübertragungskanal verwendet wird.

Bei normalem Betrieb erfolgt die Datenübertragung zwischen dem extrakorporalen Teil und dem intrakorporalen Teil der Sehprothese überwiegend mittels des Hinübertragungskanal, d.h. die von dem extrakorporalen Teil der Sehprothese (bspw. in der Brille) erfassten und bearbeiteten Bilddaten werden über den Hinübertragungskanal an den intrakorporalen Teil der Sehprothese (im Auge) übermittelt bzw. gesendet. Auf dem Rückübertragungskanal wird dagegen nur im Falle einer Rückmeldung vom intrakorporalen Teil an den extrakorporalen Teil der Sehprothese gesendet, wenn beispielsweise Daten über den Zustand des intrakorporalen Teils der Sehprothese oder eine Fehlermeldung übermittelt werden sollen.

Auf dem Hinübertragungskanal (Up-Link) von der Brille zu dem Implantat im Auge können unterschiedliche Arten von Daten übertragenen werden. Über den Hinübertragungskanal werden beispielsweise Stimulationsbefehle vom extrakorporalen Teil der Sehprothese an den Stimulator-Chip des intrakorporalen Teils im Auge übermittelt. Solche Stimulationsbefehle können die folgenden Informationen umfassen:
- Elektrodenadressen, d.h. die Adressen der im Elektrodenarray angeordneten Elektroden, die zur Stimulation der Ganglienzellen in der Retina des Auges dienen,
- Stromamplituden, d.h. die Information für den Stimulator-Chip, welche die Stromstärke der zu erzeugenden Stimulationsimpulse angeben,
- Phasendauer, d.h. die Information für den Stimulator-Chip, welche die Phasendauer der zu erzeugenden Stimulationsimpulse beinhalten,
- Phasenverhältnis, d.h. die Information für den Stimulator-Chip, welche das Phasenverhältnis der zu erzeugenden Stimulationsimpulse angeben,
- Vorzeichen der Stimulationsstrompulse, d.h. die Information für den Stimulator-Chip, welche das Vorzeichen der zu erzeugenden Stimulationsimpulse beinhalten.

Ferner können über den Hinübertragungskanal vom extrakorporalen Teil der Sehprothese an den intrakorporalen Teil beispielsweise Messbefehle übermittelt werden. Messbefehle sind Befehle vom extrakorporalen Teil der Sehprothese an den intrakorporalen Teil, bestimmte Messungen durchzuführen und den ermittelten Messwert über den Rückübertragungskanal an den extrakorporalen Teil der Sehprothese zu übermitteln. Solche Messbefehle können die folgenden Informationen umfassen:
- Spannungsmessung an einer Elektrode während der Stimulation,
- Spannungsmessung an einer Elektrode außerhalb der Stimulation,
- Messung von Nerven-Aktions-Potentialen mit Hilfe einer oder mehrerer Stimulationselektroden,
- Messung von Nerven-Aktions-Potentialen mit Hilfe spezieller Messelektroden.

Des weiteren können über den Hinübertragungskanal vom extrakorporalen Teil an den intrakorporalen Teil der Sehprothese beispielsweise Statusbefehle übermittelt werden. Solche Statusbefehle enthalten Aufforderungen an den intrakorporalen Teil der Sehprothese, bestimmte Zustandsparameter zu erfassen und über den Rückübertragungskanal an den extrakorporalen Teil der Sehprothese zu übermitteln. Die Statusbefehle können beispielsweise Aufforderungen an den intrakorporalen Teil der Sehprothese enthalten, die folgenden Zustandsparameter zu erfassen und auf dem Rückübertragungskanal an den extrakorporalen Teil der Sehprothese zu übermitteln:
- die Identifikations-Nummer(ID-Nummer) des Implantats,
- eine Status-Übersicht des Implantats, z.B.
   o über den Zustand der Ladungs-Ausgleichssysteme oder
   o über den Zustand der Energieversorgung des Implantats, d.h. ob diese beispielsweise zu viel Energie oder zu wenig Energie aufweist,
- die Temperatur des Stimulations-Chips oder von Implantat-Teilen,
- der Feuchtigkeitssensor-Messwert.

Gemäß einer weiteren bevorzugten Ausführungsform der erfindungsgemäßen Sehprothese ist zumindest der Hinübertragungskanal vom extrakorporalen Teil an den intrakorporalen Teil der Sehprothese als optische Datenübertragung ausgestaltet. Dabei können die Daten durch Lichtsignale mittels lichtemittierender Dioden (LED) oder mittels Laser, beispielsweise mit Infrarotlicht übertragen werden. Bei der optischen Datenübertragung kann zumindest teilweise der natürliche Lichtweg des Auges benutzt werden, indem die Lichtsignale der lichtemittierenden Dioden oder des Lasers außerhalb des Auges durch die Linsenöffnung des Auges auf ein optisches Empfangselement im Inneren des Auges gerichtet werden.

Die Datenübertragung zwischen dem intrakorporalen Teil und dem extrakorporalen Teil der Sehprothese kann auf dem Rückübertragungskanal mit beliebiger Kodierung geschehen. Vorzugsweise wird eine ausgeglichene Kodierung verwendet, die in etwa gleich viele Null-Zustände und Eins-Zustände enthält, um das optische Empfangselement nicht in eine Sättigung zu treiben. Es können beispielsweise die Manchester-Kodierung, die sogenannte 4PPM Kodierung, die 4PPM+ Kodierung oder andere geeignete Kodierungs-Verfahren verwendet werden.

Gemäß einer weiteren bevorzugten Ausführungsform der erfindungsgemäßen Sehprothese ist der Hin- und/oder der Rückübertragungskanal zwischen dem intrakorporalen Teil und dem extrakorporalen Teil der Sehprothese als elektromagnetische Datenübertragung ausgestaltet, bei dem die Trägerfrequenz des Senders entsprechend moduliert wird, um Daten zu übertragen. Dabei kann die elektromagnetische Datenübertragung aktiv ausgebildet sein, wobei als Trägerfrequenz des Senders beispielsweise das 13,56 MHz ISM Frequenz-Band, das 27,12 MHz ISM Frequenz-Band, das 125 kHz ISM Frequenz-Band oder ein anderes geeignetes Frequenz-Band verwendet wird. Bei der elektromagnetischen Datenübertragung zwischen dem intrakorporalen Teil und dem extrakorporalen Teil der Sehprothese können anstelle einer Frequenz-Modulation auch eine Amplituden-Modulation, eine Phasen-Modulation der Trägerfrequenz oder andere geeignete Modulationsverfahren angewendet werden.

Gemäß einem weiteren für die erfindungsgemäße Sehprothese anwendbaren Modulationsverfahren wird eine separate Datenträger-Frequenz, z.B. im 433 MHz ISM Frequenz-Band oder in einem anderen geeigneten Frequenzbereich verwendet, die vorzugsweise von der Frequenz zur Energieeinkopplung über die induktive Schnittstelle verschieden ist. Diese separate Datenträger-Frequenz kann wiederum mit den folgenden Methoden moduliert werden:
- Amplitudenmodulation der Datenträgerfrequenz,
- Frequenzmodulation der Datenträgerfrequenz,
- Phasenmodulation der Datenträgerfrequenz ,
- andere geeignete Modulationsverfahren.

Über den Rückübertragungskanal (Down-Link) vom intrakorporalen Teil zum extrakorporalen Teil der Sehprothese können unterschiedliche Arten von Daten übertragen werden. Durch den Rückübertragungskanal können insbesondere Diagnosedaten über den Zustand des intrakorporalen Teils der Sehprothese bzw. über den Zustand des Implantats übermittelt werden, wie z.B.:
- Messwerte zur Elektrodenimpedanz bestimmter Stimulationselektroden,
- Messwerte zur elektrischen Spannung, die an Stimulationselektroden anliegt,
- Überwachungsdaten des Zustands bestimmter Stimulationselektroden.

Durch den Rückübertragungskanal können auch Informationen bzw. Diagnosedaten über den Systemstatus der Ablaufsteuerung im intrakorporalen Teil der Sehprothese bzw. im Implantat übermittelt werden, wie z.B. Informationen zu den folgenden Steuerungsdetails:
- Daten vom extrakorporalen Teil an den intrakorporalen Teil der Sehprothese korrekt übermittelt (ja oder nein),
- intrakorporaler Teil bzw. Implantat korrekt initialisiert (ja oder nein),
- Zurücksetzen des Systemstatus (Reset) durchgeführt (ja oder nein),
- Status zur Energieversorgung des Implantats (Power-Status), z.B.
   o Status des oder der analogen Komponenten des Stimulator-Chips,
   o Zustand der sogenannten Power-Down-Stufe des intrakorporalen Teils an den extrakorporalen Teil der Sehprothese senden,
- Fehler bei der Stimulation der Retina des Auges, z.B.
   o maximaler Stimulationsstrom erreicht,
   o Ladungsausgleich zwischen Stimulationselektroden nicht erreicht,
   o Ladungsausgleich zwischen Stimulationselektroden dauert zu lange,
- Stimulation wurde durchgeführt, obwohl nicht angefordert, z.B. durch einen Fehler in Endstufe einer Stromquelle,
- Status der elektrischen Energieversorgung, z.B.
   o Betriebsspannung zu gering,
   o Betriebsspannung zu hoch,
- Spannung an Stimulationselektroden zu bestimmten Messzeitpunkten.
- Diagnosedaten zur Physiologie des Patienten, z.B.
   o Ableitung der Aktionspotentiale einzelner Nervenzellen, insbesondere Ganglienzellen,
   o Ableitung der Summen-Aktionspotentiale von Nervenzellen, insbesondere Ganglienzellen, wodurch eine Aussage über die Stimulierbarkeit der kontaktierten Nervenzellen möglich ist,
- Diagnosedaten zum allgemeinen Status des intrakorporalen Teils der Sehprothese bzw. des Implantats, z.B.
   o Temperatur im Bereich der Elektronik des Implantats,
   o Temperatur in einem bestimmten Punkt des Auges,
   o Temperatur an mehreren Stellen des Auges,
   o Messwert zum Augeninnendruck,
   o Beschleunigungsmessung des Implantats,
   o Feuchtigkeitsmessung innerhalb des Gehäuses des Implantats,

Wie oben bereits im Zusammenhang mit dem Hinübertragungskanal beschrieben, kann auch der Rückübertragungskanal vom intrakorporalen Teil an den extrakorporalen Teil der Sehprothese als optischer Datenübertragungsweg ausgebildet sein. Dazu können die Daten wie bei dem Hinübertragungskanal so auch beim Rückübertragungskanal durch Lichtsignale mittels lichtemittierender Dioden (LED) oder mittels Laser, beispielsweise mit Infrarotlicht übertragen werden. Dabei kann ebenfalls der natürliche Lichtweg des Auges zumindest teilweise benutzt werden, indem die Lichtsignale eines im Inneren des Auges angeordneten lichtemittierenden Elements durch die Linsenöffnung des Auges auf ein optisches Empfangselement außerhalb des Auges gerichtet werden. Das optische Empfangselement erfasst die kodierten Lichtsignale des lichtemittierenden Elements und leitet diese in Form von elektrischen Signalen an eine Elektronik zur Auswertung weiter.

Gemäß einer weiteren bevorzugten Ausführungsform der erfindungsgemäßen Sehprothese ist der Rückübertragungskanal vom intrakorporalen Teil zum extrakorporalen Teil der Sehprothese als passive elektromagnetische Datenübertragung ausgestaltet, bei dem die Trägerfrequenz eines Senders entsprechend moduliert wird, um Daten zu übertragen. Dabei kann als Modulationsverfahren beispielsweise eine Lastmodulation der Energie-Sendefrequenz durchgeführt werden. Die Last-Modulation der Trägerfrequenz kann durch Zuschalten und Abschalten einer resistiven Last, einer kapazitiven Last oder einer induktiven Last entsprechend dem zu übertragenen Datenstrom erfolgen. Es ist auch eine Kombination oder Teilkombination der genannten Verfahren bei der elektromagnetischen Datenübertragung zwischen dem intrakorporalen Teil und dem extrakorporalen Teil der Sehprothese möglich.

Die Datenübertragung über den Rückübertragungskanal vom intrakorporalen Teil (im Auge) zum extrakorporalen Teil (bspw. in der Brille) der erfindungsgemäßen Sehprothese kann zusätzlich oder alternativ unter Anwendung eines Verfahrens zur Fehlerkorrektur durchgeführt werden. Ebenso kann auch die Datenübertragung über den Hinübertragungskanal vom extrakorporalen Teil (in der Brille) zum intrakorporalen Teil der Sehprothese (im Auge) zusätzlich oder alternativ unter Anwendung eines Verfahrens zur Fehlerkorrektur durchgeführt werden.

Als Fehlerkorrekturverfahren kann beispielsweise ein Verfahren angewendet werden, das durch Redundanz in der Kodierung der Datenübertragung eine definierte Anzahl von falsch übertragenen Datenbits pro insgesamt übertragenen Datenbits wieder korrigieren kann. Als Verfahren zur Fehlerkorrektur kann sowohl für den Hinübertragungskanal als auch für den Rückübertragungskanal eines der folgenden Verfahren verwendet werden:
- Hamming-Kodierung,
- Faltungs-Kodierung,
- Wiederholungs-Kodierung oder
- andere geeignete Fehlerkorrekturverfahren.

Zusätzlich oder alternativ zur Anwendung eines Fehlerkorrekturverfahren kann die Datenübertragung über den Hinübertragungskanal vom extrakorporalen Teil (in der Brille) zum intrakorporalen Teil der Sehprothese (im Auge) unter Anwendung eines Verfahrens zur Fehlerdetektion durchgeführt werden. Ebenso kann auch die Datenübertragung über den Rückübertragungskanal vom intrakorporalen Teil (im Auge) zum extrakorporalen Teil (in der Brille) der erfindungsgemäßen Sehprothese zusätzlich oder alternativ zum Verfahren zur Fehlerkorrektur unter Anwendung eines Verfahrens zur Fehlerdetektion durchgeführt werden. Ein solches Verfahren zur Fehlerdetektion kann durch unterschiedliche Kodierungs-Verfahren implementiert werden, wie z.B.
- die Cyclic Redundancy Check (CRC) Kodierung,
- die Parity Check-Kodierung,
- die Wiederholungs-Kodierung oder
- andere geeignete Fehlerdetektionsverfahren.

Für die Datenübertragung über den Hinübertragungskanal vom extrakorporalen Teil (in der Brille) zum intrakorporalen Teil (im Auge) der erfindungsgemäßen Sehprothese kann eine Datenrate im Bereich von 100 Kilo-Bit/Sekunde bis 10 MegaBit/Sekunde, vorzugsweise eine Datenrate im Bereich von 1 MegaBit/Sekunde bis 10 MegaBit/Sekunde besonders bevorzugt von 1 bis 5 MegaBit/Sekunde und besonders bevorzugt von 1 bis 2 MegaBit/Sekunde verwendet werden.

Für die Datenübertragung über den Rückübertragungskanal vom intrakorporalen Teil (im Auge) zum extrakorporalen Teil (in der Brille) der erfindungsgemäßen Sehprothese kann eine Datenrate im Bereich von 1 KiloBit/Sekunde bis 100 KiloBit/Sekunde, vorzugsweise eine Datenrate im Bereich von 5 bis 20 KiloBit/Sekunde verwendet werden. Die zur Datenübertragung über den Hinübertragungskanal und zur Datenübertragung über den Rückübertragungskanal verwendeten Datenraten können dabei unterschiedlich sein.

Weitere Einzelheiten, bevorzugte Ausführungsformen und Vorteile der vorliegenden Erfindung ergeben sich aus der nachfolgenden Beschreibung unter Bezugnahme auf die Zeichnung. Es zeigen:
- Figur 1: eine schematische Darstellung vom Querschnitt durch ein menschliches Auge mit einer Sehprothese gemäß einer bevorzugten Ausführungsform der vorliegenden Erfindung;
- Figur 2: eine perspektivische Ansicht eines Stimulations-Systems umfassend eine Brille und ein menschliches Auge mit einer erfindungsgemäßen Sehprothese;
- Figur 3: eine schematische Darstellung vom Querschnitt durch ein menschliches Auge mit einer Sehprothese gemäß einer zweiten bevorzugten Ausführungsform der vorliegenden Erfindung;
- Figur 4: eine schematische Darstellung vom Querschnitt durch ein menschliches Auge mit einer Sehprothese gemäß einer dritten bevorzugten Ausführungsform der vorliegenden Erfindung;
- Figur 5: eine schematische Darstellung vom Querschnitt durch ein menschliches Auge mit einer Sehprothese gemäß einer vierten bevorzugten Ausführungsform der vorliegenden Erfindung; und
- Figur 6: eine schematische Darstellung vom Querschnitt durch ein menschliches Auge mit einer Sehprothese gemäß einer fünften bevorzugten Ausführungsform der vorliegenden Erfindung.

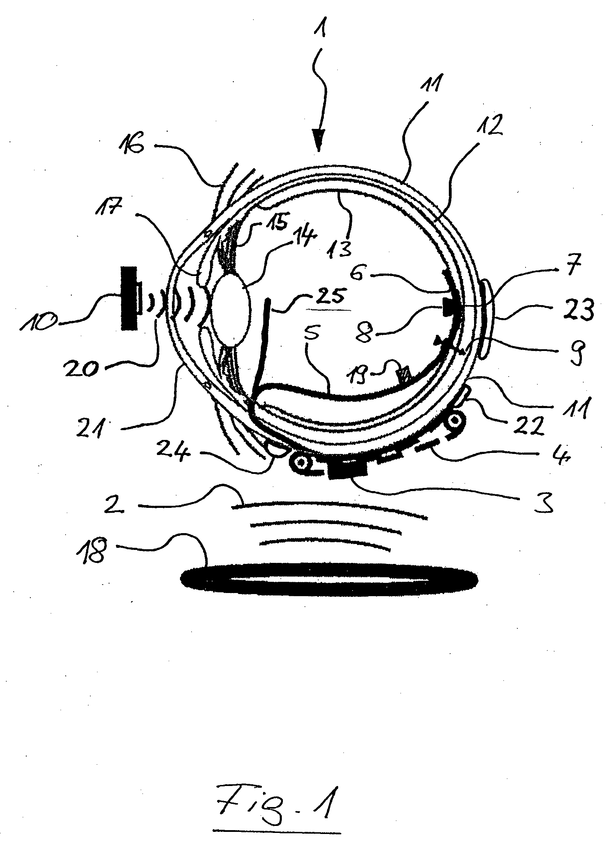

Figur 1 zeigt eine schematische Darstellung im Querschnitt durch ein menschliches Auge mit einer Sehprothese gemäß einer bevorzugten Ausführungsform der vorliegenden Erfindung. Der Augapfel 1 des menschlichen Auges hat eine im wesentlichen runde Form, wobei die transparente Hornhaut 21 an dessen Vorderseite eine stärkere Krümmung aufweist. Der in der Augenhöhle gelagerte Bereich des Augapfels 1 ist aus mehreren Lagen aufgebaut, wobei die äußerste Lage die sogenannte Sklera bzw. Lederhaut 11 darstellt. An die Lederhaut bzw. Sklera 11 schließt sich in Richtung des Augeninneren die Aderhaut 12 an, auf der die sogenannte Retina bzw. Netzhaut 13 mit lichtempfindlichen Zellen bzw. Photorezeptoren (Zapfen, Stäbchen und Ganglienzellen) aufliegt.

Bei einem gesunden menschlichen Auge erfolgt der natürliche Lichtweg über die transparente Hornhaut 21 im vorderen Bereich des Augapfels 1 durch die Regenbogenhaut 17 und die bikonvexe Linse 14, deren Form bzw. Brechkraft durch die Anspannung des Ziliarmuskels 15 veränderlich ist. Das einfallende Licht tritt unter der optischen Brechung der Hornhaut 21 und der Augenlinse 14 in das Augeninnere und wird auf die Netzhaut 13 im hinteren Bereich des Augapfels 1 projiziert. Die lichtempfindlichen Photorezeptoren in der Netzhaut 13 wandeln das auf die Netzhaut projizierte Bild des einfallenden Lichts in Nervensignale um, die durch Ganglienzellen in der Netzhaut (nicht dargestellt) an das Gehirn weitergeleitet werden.

Die Sehprothese gemäß der vorliegenden Erfindung hat zum Ziel, einen aufgrund von degenerativen Veränderungen an der Netzhaut 13 beeinträchtigten oder zerstörten Sehvorgang wieder herzustellen bzw. zu verbessern. Eine Voraussetzung für den Einsatz der erfindungsgemäßen Sehprothese ist, dass die sich in der Netzhaut 13 befindlichen Ganglienzellen weitgehend intakt und in der Lage sind, Nervenimpulse über den Sehnerv an das Gehirn weiterzuleiten.

Die erfindungsgemäße Sehprothese umfasst gemäß der in Figur 1 dargestellten bevorzugten Ausführungsform ein Stimulations-System mit einem intraokularen Implantat 6, 8, das innerhalb des Augapfels 1 angeordnet ist, und ein extraokulares Implantat 3, 4, das außerhalb des Augapfels 1 angeordnet ist. Das intraokulare Implantat ist als epiretinales Implantat ausgebildet, d.h. dass das intraokulare Implantat aus Sicht des Augeninneren auf der Netzhaut bzw. Retina 13 im hinteren Bereich des Auges angeordnet ist.

Das intraokulare Implantat ist mit dem extraokularen Implantat über eine Drahtverbindung 5 gekoppelt. Die Drahtverbindung 5 ist als ein flexibles Implantat ausgebildet, das von dem extraokularen Implantat außerhalb des Augapfels 1 unmittelbar hinter der Bindehaut 16 im Bereich der sogenannten Parsplana zwischen dem Ziliarmuskel 15 und der Netzhaut 13 in das Augeninnere zum intraokularen Implantat führt. Die Drahtverbindung 5 umfasst elektrische Leitungen, um die Stromversorgung des intraokularen Implantats über das extraokulare Implantat zu gewährleisten. Ferner umfasst die Drahtverbindung 5 elektrische Leitungen in einer ausreichenden Anzahl, um die Übermittlung von Bild-Daten bzw. Diagnosebefehlen, Steuerbefehlen oder Stimulationsbefehlen in Form von seriellen Datenströmen und/oder parallelen Datenströmen bzw. Signalströmen zwischen dem intraokularen Implantat und dem extraokularen Implantat zu ermöglichen.

Das intraokulare Implantat umfasst ein Elektrodenarray 6, das auf der Netzhaut 13 epiretinal aufliegt und eine Anzahl von beispielsweise in einer Matrix angeordneten Stimulations-Elektroden aufweist. Die Stimulations-Elektroden des Elektrodenarrays 6 stehen mit Ganglienzellen in Verbindung und können diese mittels Stimulations-Impulsen bzw. Stimulations-Strömen stimulieren. Das Elektrodenarray 6 des epiretinalen Implantats ist im Bereich der Makula des Auges zentriert, wo über den natürlichen Lichtweg das meiste Licht auf die Netzhaut 13 fällt. Um eine sichere Position des intraokularen Implantats auf der Netzhaut zu gewährleisten, ist es mit Hilfe eines sogenannten Tack bzw. Nagels 9 im Augeninneren befestigt, der sich durch das intraokulare Implantat sowie die Netzhaut 13 erstreckt und mit Widerhaken in der Lederhaut 11 verankert ist.

Auf dem intraokularen Implantat ist ein Infrarot-Empfänger 8 angeordnet, der Licht-Signale von einem Infrarot-Sender 10 außerhalb des Auges über den natürlichen Lichtweg empfangen kann. Über eine externe Kamera (nicht dargestellt) wird ein Bild erfasst, deren vorverarbeitete Bild-Daten über den Infrarot-Sender 10 auf dem natürlichen Lichtweg des menschlichen Auges an den Infrarot-Empfänger 8 des intraokularen Implantats übermittelt werden. Diese Bild-Daten werden über die Drahtverbindung 5 vom intraokularen Implantat an das extraokulare Implantat vorzugsweise in Form eines seriellen Datenstroms weitergeleitet.

Die Position des Infrarot-Empfängers 8 ist auf der gesamten Drahtverbindung 5 denkbar, er befindet sich jedoch vorzugsweise im Bereich des Nagels 9. Alternativ kann der Infrarot-Empfänger 8 auf einer Abzweigung 25 der Drahtverbindung 5 liegen, um die Empfangseigenschaften günstig einzustellen. Diese Abzweigung 25 steht von der Drahtverbindung 5 ab und ragt zweckmäßigerweise in den Strahlengang des natürlichen Lichtweges in das Auge hinein. Auf diese Weise können die von außerhalb des Auges über den natürlichen Lichtweg in das Auge einfallenden Infrarot-Signale unmittelbar auf den an der Abzweigung 25 der Drahtverbindung 5 angeordneten Infrarot-Empfänger 8 auftreffen.

Die Bild-Daten werden im Retina-Stimulator-Chip 3 des extraokularen Implantats ausgewertet und in Stimulations-Impulse bzw. Stimulations-Ströme umgewandelt. Die Stimulations-Impulse bzw. Stimulations-Ströme werden anschließend in Form eines parallelen Signalstroms über die Drahtverbindung 5 an die Stimulations-Elektroden im Elektrodenarray 6 des intraokularen Implantats übermittelt und fließen über die Gegenelektrode 22, 23 und/oder 24 zurück in die jeweilige Stromquelle. Die Stimulations-Elektroden stimulieren die Ganglienzellen in der Retina über die Mikrokontaktstruktur entsprechend den ortsaufgelösten Stimulations-Impulsen und erzeugen dadurch mit Nervensignalen einen visuellen Eindruck, der dem von der externen Kamera aufgenommenen Bild entspricht.

Das Stimulations-System der erfindungsgemäßen Sehprothese umfasst ferner ein extraokulares Implantat, das außerhalb des Augapfels 1 an der Lederhaut 11 angeordnet ist. In dem extraokularen Implantat sind alle diejenigen Komponenten des Stimulations-Systems untergebracht, die nicht zwingend am intraokularen Implantat im Augeninneren angeordnet sein müssen. Das extraokulare Implantat umfasst einen Retina-Stimulator-Chip 3, der aufgrund von empfangenen Bild-Daten Stimulations-Impulse bzw. Stimulations-Ströme für die Stimulations-Elektroden des intraokularen Implantats berechnen und generieren kann. Dazu umfasst der Retina-Stimulator-Chip 3 elektronische Bauteile zur Berechnung der Intensität und Dauer der Stimulations-Impulse anhand der empfangenen Bild-Daten, Stromgeneratoren zur Erzeugung der erforderlichen Stimulations-Ströme sowie elektronische Speichermittel, in denen die Parameter der Stimulations-Impulse und die Koordinaten der entsprechenden Stimulations-Elektroden zwischengespeichert und auf ein bestimmtes Kommando hin abgerufen werden können bzw. ausgelöst werden können.

Das extraokulare Implantat umfasst ferner mindestens eine Gegenelektrode, die z.B. in der Position angeordnet sein können, die in Figur 1 durch die Bezugszeichen 22, 23 und 24 gekennzeichnet sind. Die Gegenelektroden 22, 23, 24 dienen als Rückstromweg für die Stimulationsstromquellen, um den Strompfad zu den Stimulations-Elektroden im Elektrodenarray 6 über das Gewebe der Lederhaut 11, Aderhaut 12 und der Netzhaut 13 zu schließen.

Das extraokulare Implantat umfasst ferner eine Hochfrequenz-Antenne 4, über die Hochfrequenz-Signale 2 empfangen werden können, die von einer vom Augapfel 1 entfernt angeordneten Hochfrequenz-Antenne 18 ausgesendet wurden. Über die induktive Schnittstelle zwischen der Hochfrequenz-Antenne 4 des extraokularen Implantats und der externen Hochfrequenz-Antenne 18 kann induktiv Energie übermittelt werden, die für den Betrieb des extraokularen Implantats und des intraokularen Implantats erforderlich ist.

Die externe Hochfrequenz-Antenne 18 kann beispielsweise zusammen mit anderen Elektronik-Komponenten außerhalb des Körpers in einem extrakorporalen Teil der erfindungsgemäßen Sehprothese untergebracht sein, wie z.B. in einer Brille, die der Patient wie eine normale Sehhilfe tragen kann. Demgegenüber stellen das intraokulare Implantat 6, 8 und das extraokulare Implantat 3, 4 einen intrakorporalen Teil 3, 4, 6, 8 der erfindungsgemäßen Sehprothese dar. Über die induktive Schnittstelle kann zwischen dem extrakorporalen Teil und dem intrakorporalen Teil der erfindungsgemäßen Sehprothese ein drahtloser Kontakt hergestellt werden.

Über diese induktive Schnittstelle zwischen dem extrakorporalen Teil und dem intrakorporalen Teil können auch die von einer externen Kamera aufgenommene Bild-Daten an den Retina-Stimulator-Chip 3 übermittelt werden, der anhand der empfangenen Bild-Daten Stimulations-Impulse erzeugt und über die Drahtverbindung 5 an die Stimulations-Elektroden im intraokularen Implantat weiterleitet. Zusätzlich kann die induktive Schnittstelle zwischen der Hochfrequenz-Antenne des intraokularen Implantats und der externen Hochfrequenz-Antenne 18 bidirektional ausgebildet sein, sodass der Retina-Stimulator-Chip 3 Informationen über Betriebsparameter des intraokularen Implantats und/oder des extraokularen Implantats induktiv über die Hochfrequenz-Antenne 4 an die externe Hochfrequenz-Antenne 18 übermitteln kann, die anschließend von einer externen Elektronik (nicht dargestellt) ausgewertet werden können.

Zur Einrichtung der bidirektionalen induktiven Schnittstelle zwischen dem extrakorporalen Teil und dem intrakorporalen Teil der erfindungsgemäßen Sehprothese kann der extrakorporale Teil außerhalb des Auges 1 eine Antenne 18 aufweisen, die elektromagnetische Signale 2 vorzugsweise im Hochfrequenz-Bereich sowohl senden als auch empfangen kann. Ebenso kann das extraokulare Implantat 3, 4 und/oder das intraokulare Implantat 6, 8 eine Antenne 4 aufweisen, die elektromagnetische Signale 2 vorzugsweise im Hochfrequenz-Bereich sowohl senden als auch empfangen kann.

Alternativ kann der extrakorporale Teil der erfindungsgemäßen Sehprothese mindestens zwei Antennen 18 für die bidirektionale induktive Schnittstelle umfassen, von denen eine erste Antenne elektromagnetische Signale 2 senden kann und eine zweite Antenne elektromagnetische Signale 2 empfangen kann. Ebenso kann der extrakorporale Teil der erfindungsgemäßen Sehprothese, d.h. das extraokulare Implantat 3, 4 und/oder das intraokulare Implantat 6, 8, mindestens zwei Antennen 4 für die bidirektionale induktive Schnittstelle umfassen, von denen eine erste Antenne elektromagnetische Signale 2 senden kann und eine zweite Antenne elektromagnetische Signale 2 empfangen kann.

Das intraokulare Implantat umfasst ferner ein lichtemittierendes Element 19, das Licht-Signale in Abhängigkeit von Betriebsparametern des intraokularen Implantats erzeugt. Dieses lichtemittierende Element 19 ist beispielsweise als Infrarot-Diode ausgebildet, deren Infrarot-Lichtsignale von einem Beobachter oder einem entsprechenden Infrarot-Empfänger außerhalb des Auges wahrgenommen werden können. Mit Hilfe der von dem lichtemittierenden Element 19 ausgesendeten Licht-Signale lässt sich beispielsweise die optimale Position des intraokularen Implantats auf der Netzhaut 13 während der operativen Implantation feststellen. Das lichtemittierende Element 19 kann deshalb auch als Status-Anzeige bezeichnet werden. Die Position des lichtemittierenden Elements 19 ist auf dem gesamten Bereich der Drahtverbindung 5 denkbar, es befindet sich jedoch vorzugsweise im Bereich des Nagels 9. Alternativ kann das lichtemittierende Element 19 auf der Abzweigung 25 der Drahtverbindung 5 liegen, um die Abstrahleigenschaften günstig einzustellen.

Zur Übertragung von Informationen können bei der Datenübertragung über die bidirektionale induktive Schnittstelle die elektromagnetischen Signale 2 und bei der Datenübertragung über die optische Schnittstelle die Licht-Signale unter Anwendung einer der oben beschriebenen Verfahren kodiert werden. Dabei können auch die oben beschriebenen Verfahren zur Fehler-Korrektur und zur Fehler-Detektion angewendet werden.

Die vorliegende Erfindung löst die oben genannte Aufgabe durch eine Sehprothese mit einem epiretinalen Implantat, das über eine extraokulare Vorrichtung mit Strom versorgt wird, wobei die extraokulare Vorrichtung den Strom über eine induktive Schnittstelle und damit drahtlos von einem Hochfrequenzsender empfängt. Der Hochfrequenzsender kann innerhalb oder nahe der Augenhöhle beispielsweise in einer Brille angeordnet sein oder auch von dem mit dem Implantat versehenen menschlichen Auge entfernt.

Ferner löst die vorliegende Erfindung die oben genannte Aufgabe durch eine bidirektionale induktive Schnittstelle zwischen einer außerhalb des Auges und des Körpers angeordneten Senders/Empfängers bzw. Antenne und einer innerhalb des Körpers am oder im Auge angeordneten Senders/Empfängers bzw. Antenne, über die eine bidirektionale Datenübertragung zwischen dem extrakorporalen Teil und dem intrakorporalen Teil der Sehprothese erfolgen kann.

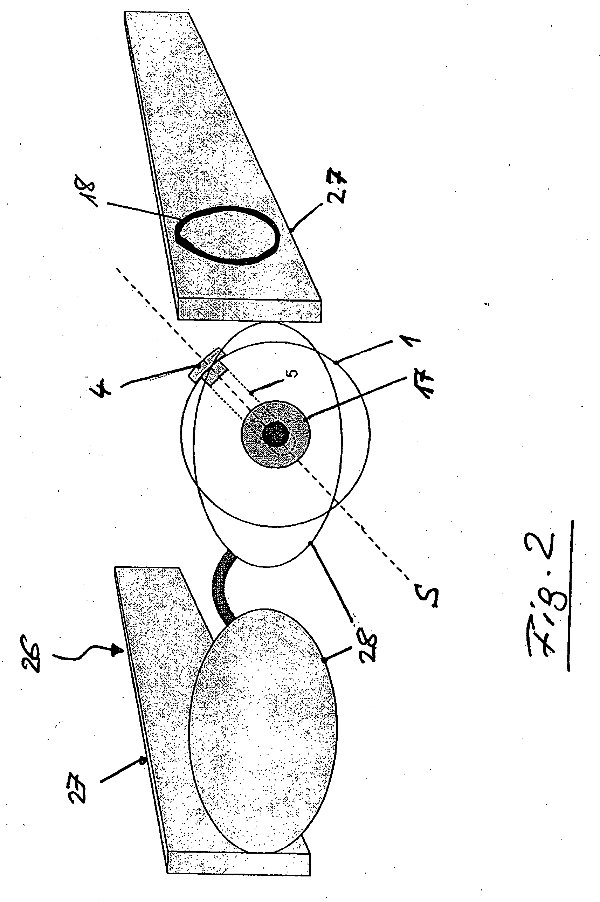

Figur 2 zeigt eine perspektivische Ansicht eines Stimulations-Systems umfassend eine Brille und ein menschliches Auge mit einer erfindungsgemäßen Sehprothese. Bei dem in Figur 2 dargestellten Stimulations-System sind die extrakorporalen Komponenten der erfindungsgemäßen Sehprothese in einer Brille bzw. einem Brillengestell 26 untergebracht, die der Patient wie eine gewöhnliche Brille tragen kann. Die Brille 26 umfasst zwei Brillenbügel 27 zur üblichen Anordnung der Brille 26 am Kopf des Patienten und zwei Brillenglasrahmen 28 zur Aufnahme von Brillengläsern, die ohne optische Funktion sein können und lediglich dem natürlichen Aussehen der Brille dienen.

In dem Brillenbügel 27 kann beispielsweise die externe Kamera, insbesondere eine Videokamera (nicht dargestellt) untergebracht sein, die das Bild bzw. aufeinander folgende Sequenzen von Bildern vor dem Gesichtsfeld des Patienten erfasst. Elektronische Komponenten der Sehprothese, die zur Bearbeitung und Aufbereitung der von der externen Kamera aufgenommenen Bild-Daten erforderlich sind, können ebenfalls in der Brille bzw. im Brillengestell 26 untergebracht werden. Zusätzlich oder alternativ können elektronische Komponenten der Sehprothese in einem sogenannten Pocket-Computer untergebracht werden, den der Patienten in einer separaten Tasche am Körper transportieren kann.

In der Brille 26, insbesondere in den Brillenbügeln 27 können auch die Empfänger-Spule und die Sende-Spule 18 des extrakorporalen Teils der Sehprothese untergebracht sein, die elektromagnetische Signale vorzugsweise im Hochfrequenz-Bereich senden bzw. empfangen können. Aufgrund der Sende- und Empfangsfunktionen der Sende- bzw. Empfänger-Spule 18 in der Brille und der Sende- bzw. Empfänger-Spule 4 des extraoklularen, intrakorporalen Teils der Sehprothese ist die induktive Schnittstelle zwischen dem extrakorporalen Teil und dem intrakorporalen Teil der Sehprothese bidirektional ausgebildet. Vorteilhafterweise sind die Empfänger-Spule und/oder die Sende-Spule 18 bzw. die Sender/Empfänger-Spule des extrakorporalen Teils der Sehprothese im Brillenglasrahmen 28 untergebracht, indem beispielsweise die Schleife des Brillenglasrahmens 28 die Spule selbst darstellt.

Bei der in Figur 2 dargestellten Ausführungsform des erfindungsgemäßen Stimulations-Systems wird im Betrieb zunächst von der externen Kamera in der Brille 26 eine Bildaufnahme vorgenommen, deren Bild-Signale nach einer elektronischen Vorverarbeitung über die Sende- bzw. Empfänger-Spule 18 im Brillenbügel 27 an die Sende- bzw. Empfänger-Spule 4 des intrakorporalen Teils induktiv übertragen werden und von dort über die Drahtverbindung 5 an das epiretinale Elektrodenarray 8 des intraokularen Implantats weitergeleitet werden. Das Elektrodenarray 8, stimuliert die Zellen der Retina durch elektrische Signale entsprechend den empfangenen Bild-Daten und gibt damit das von der externen Kamera aufgenommene Bild an die Nerven des visuellen Systems weiter. Auf diese Weise wird das durch die Kamera erfasste Bild in elektrische Signale umgesetzt, vom extrakorporalen Teil über die bidirektionale induktive Schnittstelle an den intrakorporalen Teil der Sehprothese übertragen und über Stimulations-Elektroden mittels elektrischer Stimulations-Impulse an die Ganglienzellen der Retina bzw. an den Sehnerv abgegeben, um so das Sehvermögen eines sehbehinderten Patienten wiederherzustellen bzw. zu verbessern.

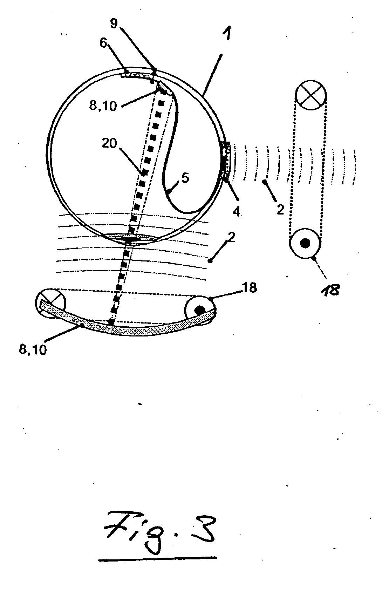

In Figur 2 ist auch eine gestrichelte Linie S dargestellt, die zentrisch durch das Auge 1 verläuft und die Schnittebene der Figuren 3 bis 6 darstellt. Figur 3 zeigt eine schematische Darstellung vom Querschnitt entlang der in Figur 2 gezeigten Schnittebene S durch ein menschliches Auge mit einer Sehprothese gemäß einer zweiten bevorzugten Ausführungsform der vorliegenden Erfindung. Bei dieser zweiten bevorzugten Ausführungsform der erfindungsgemäßen Sehprothese umfasst der intraokulare Teil das Elektrodenarray bzw. die Mikrokontaktstruktur 6, den Nagel bzw. Tack 9 zu deren epiretinalen Befestigung, den Infrarot-Empfänger 8 und die Drahtverbindung 5 zwischen dem intraokularen Teil und den extraokularen Komponenten der Sehprothese. Als extraokulare, intrakorporale Komponente der Sehprothese ist eine Sender/Empfänger-Spule 4 dargestellt, die elektromagnetische Wellen 2 sowohl senden als auch empfangen kann.

Unterhalb des Auges 1 ist eine Sender-Spule 18 angeordnet, die sich außerhalb des Körpers befindet und über elektromagnetische Wellen 2 vorzugsweise im Hochfrequenzbereich Signale an die Sender/Empfänger-Spule 4 induktiv übermittelt. Die von der extraokularen Sender/Empfänger-Spule 4 empfangenen Signale werden dann über die Drahtverbindung 5 an den intraokularen Teil der Sehprothese weitergeleitet, wie oben beschrieben. Auf der rechten Seite des Auges ist eine Empfänger-Spule 18 angeordnet, die sich ebenfalls außerhalb des Körpers befindet und die von der extraokularen Sender/Empfänger-Spule 4 ausgestrahlten elektromagnetischen Signale 2 induktiv empfängt. Auf diese Weise können Signale oder Daten induktiv von außerhalb des Auges 1 an den intraokularen Teil der Sehprothese übermittelt werden sowie umgekehrt Signale oder Daten induktiv von innerhalb des Auges 1 an den extrakorporalen Teil der Sehprothese parallel übertragen werden, wie oben beschrieben.

Zusätzlich kann innerhalb des Auges 1 ein Infrarot-Sender/Empfänger 8, 10 vorgesehen sein, der Daten vom intraokularen Teil der Sehprothese durch Infrarot-Signale 20 übermittelt, die über den natürlichen Lichtweg des Auges durch die Pupille nach außen abstrahlen und von einem außerhalb des Körpers angeordneten Infrarot-Empfänger 8 aufgenommen werden. Ferner kann der extrakorporale Infrarot-Empfänger 8 auch die Funktion eines Infrarot-Senders haben oder zu dem Infrarot-Empfänger 8 kann ein separater Infrarot-Sender 10 vorgesehen sein, der Daten durch Infrarot-Signale 20 übermittelt, die von außerhalb des Körpers über den natürlichen Lichtweg des Auges durch die Pupille in das Auge hineinstrahlen und vom Infrarot-Sender/Empfänger 8, 10 des intraokularen Teils der Sehprothese aufgenommen werden. Auf diese Weise können Signale oder Daten mittels Infrarot-Signale 20 von außerhalb des Auges 1 an den intraokularen Teil der Sehprothese übermittelt werden sowie umgekehrt Signale oder Daten mittels Infrarot-Signale 20 von innerhalb des Auges 1 an den extrakorporalen Teil der Sehprothese übertragen werden.

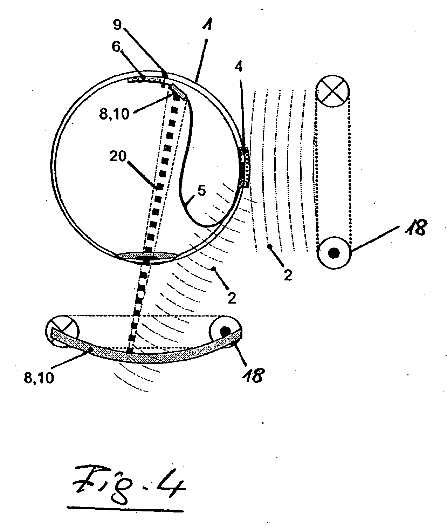

Figur 4 zeigt eine schematische Darstellung vom Querschnitt entlang der in Figur 2 gezeigten Schnittebene S durch ein menschliches Auge mit einer Sehprothese gemäß einer dritten bevorzugten Ausführungsform der vorliegenden Erfindung. Diese dritte bevorzugte Ausführungsform der erfindungsgemäßen Sehprothese umfasst ebenso wie die in Figur 3 gezeigte Ausführungsform einen intraokularen Teil mit Elektrodenarray bzw. Mikrokontaktstruktur 6, dem Nagel bzw. Tack 9, den Infrarot-Sender/Empfänger 8, 10 und die Drahtverbindung 5 zwischen dem intraokularen und dem extraokularen Teil der Sehprothese. Als extraokulare Komponente der Sehprothese ist wiederum eine Sender/Empfänger-Spule 4 dargestellt, die elektromagnetische Wellen 2 senden und empfangen kann.

Im Unterschied zu der in Figur 3 dargestellten Sehprothese ist bei der in Figur 4 gezeigten dritten Ausführungsform auf der rechten Seite des Auges eine Sender-Spule 18 angeordnet, die sich außerhalb des Körpers befindet und über elektromagnetische Wellen 2 Signale an die intrakorporale Sender/Empfänger-Spule 4 induktiv übermittelt. Die von der intrakorporalen Sender/Empfänger-Spule 4 empfangenen Signale werden dann über die Drahtverbindung 5 an den intraokularen Teil der Sehprothese weitergeleitet. Unterhalb des Auges 1 ist eine Empfänger-Spule 18 angeordnet, die sich ebenfalls außerhalb des Körpers befindet und die von der extraokularen Sender/Empfänger-Spule 4 ausgestrahlten elektromagnetischen Signale 2 induktiv empfängt. Auf diese Weise können Signale oder Daten induktiv von außerhalb des Auges 1 an den intraokularen Teil der Sehprothese übermittelt werden sowie umgekehrt Signale oder Daten induktiv von innerhalb des Auges 1 an den extrakorporalen Teil der Sehprothese in parallelem Betrieb übertragen werden, wie oben beschrieben.

Wie bei der in Figur 3 dargestellten Sehprothese kann auch bei der in Figur 4 gezeigten dritten bevorzugten Ausführungsform innerhalb des Auges 1 ein Infrarot-Sender/Empfänger 8, 10 vorgesehen sein, der Daten vom intraokularen Teil der Sehprothese durch Infrarot-Signale 20 übermittelt, die über den natürlichen Lichtweg des Auges nach außen abstrahlen und von einem außerhalb des Körpers angeordneten Infrarot-Empfänger 8 aufgenommen werden. Ferner kann der extrakorporale Infrarot-Empfänger 8 auch die Funktion eines Infrarot-Senders haben oder es kann ein separater Infrarot-Sender 10 vorgesehen sein, der Daten durch Infrarot-Signale 20 übermittelt, die von außerhalb des Körpers über den natürlichen Lichtweg des Auges in das Auge gelangen und dort vom intraokularen Infrarot-Sender/Empfänger 8, 10 aufgenommen werden. Auf diese Weise können Signale oder Daten mittels Infrarot-Signale 20 von außerhalb des Auges 1 an den intraokularen Teil der Sehprothese übermittelt werden sowie umgekehrt Signale oder Daten mittels Infrarot-Signale 20 von innerhalb des Auges 1 an den extrakorporalen Teil der Sehprothese übertragen werden.

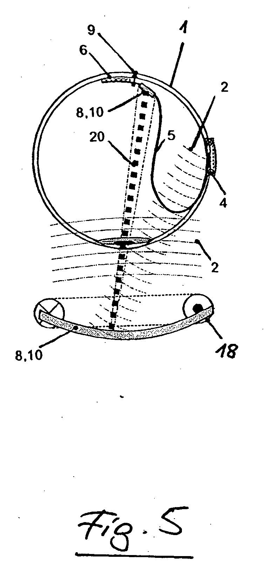

Figur 5 zeigt eine schematische Darstellung vom Querschnitt entlang der in Figur 2 dargestellten Schnittebene S durch ein menschliches Auge mit einer Sehprothese gemäß einer vierten bevorzugten Ausführungsform der vorliegenden Erfindung. Wie bei den zuvor beschriebenen Ausführungsformen ist bei dieser vierten bevorzugten Ausführungsform innerhalb des Auges 1 das Elektrodenarray 6, der Tack 9, der Infrarot-Sender/Empfänger 8, 10 und die Drahtverbindung 5 zwischen dem intraokularen Teil und den extraokularen Komponenten der Sehprothese dargestellt. Die Sender/Empfänger-Spule 4 ist wiederum intrakorporal, aber außerhalb des Auges 1 angeordnet und kann elektromagnetische Wellen 2 sowohl senden als auch empfangen.

Unterhalb des Auges 1 ist eine Sender/Empfänger-Spule 18 angeordnet, die sich außerhalb des Körpers befindet und über elektromagnetische Wellen 2 Signale an die Sender/Empfänger-Spule 4 induktiv übermittelt. Die von der extraokularen Sender/Empfänger-Spule 4 empfangenen Signale werden dann über die Drahtverbindung 5 an den intraokularen Teil der Sehprothese weitergeleitet. Im Unterschied zu den zuvor beschriebenen Ausführungsformen ist bei dieser vierten bevorzugten Ausführungsform keine separate Empfänger-Spule vorgesehen, sondern die Sender/Empfänger-Spule 18 kann ebenso wie die extraokulare Sender/Empfänger-Spule 4, elektromagnetische Wellen 2 sowohl senden als auch empfangen. Über diese bidirektionale Schnittstelle zwischen der Sender/Empfänger-Spule 4 und der Sender/Empfänger-Spule 18 können Signale oder Daten induktiv von außerhalb des Auges 1 an den intraokularen Teil der Sehprothese übermittelt werden sowie umgekehrt Signale oder Daten induktiv von innerhalb des Auges 1 an den extrakorporalen Teil der Sehprothese in alterierender Betriebsweise übertragen werden, wie oben beschrieben.

Auch bei der in Figur 5 dargestellten vierten bevorzugten Ausführungsform ist ein Infrarot-Sender/Empfänger 8, 10 innerhalb des Auges 1 vorgesehen, der Daten vom intraokularen Teil der Sehprothese durch Infrarot-Signale 20 übermittelt, die über den natürlichen Lichtweg des Auges nach außen abstrahlen und von einem außerhalb des Körpers angeordneten Infrarot-Empfänger 8 aufgenommen werden. Ferner kann der extrakorporale Infrarot-Empfänger 8 auch die Funktion eines Infrarot-Senders haben oder zu dem Infrarot-Empfänger 8 kann ein separater Infrarot-Sender 10 vorgesehen sein, der Daten durch Infrarot-Signale 20 übermittelt, die von außerhalb des Körpers über den natürlichen Lichtweg des Auges in das Auge gelangen und vom Infrarot-Sender/Empfänger 8, 10 des intraokularen Teils der Sehprothese aufgenommen werden. Auf diese Weise können Signale oder Daten mittels Infrarot-Signale 20 von außerhalb des Auges 1 an den intraokularen Teil der Sehprothese übermittelt werden sowie umgekehrt Signale oder Daten mittels Infrarot-Signale 20 von innerhalb des Auges 1 an den extrakorporalen Teil der Sehprothese übertragen werden.

Gemäß der in den Figuren 3, 4 und 5 gezeigten Ausführungsformen können der Infrarot-Sender/Empfänger 8, 10 und die extrakorporale Sender/Empfänger-Spule 18 miteinander in einer Vorrichtung kombiniert werden. Der Infrarot-Sender/Empfänger 8, 10 umfasst vorzugsweise eine parabolförmige lichtempfindliche Fläche, um die vom intraokularen Infrarot-Sender 10 über den natürlichen Lichtweg des Auges 1 nach außen gelangenden Infrarot-Signale 20 zuverlässig erfassen zu können.

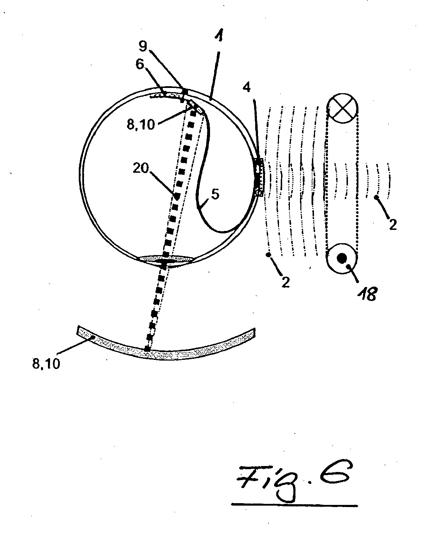

Figur 6 zeigt eine schematische Darstellung vom Querschnitt entlang der in Figur 2 gezeigten Schnittebene S durch ein menschliches Auge mit einer Sehprothese gemäß einer fünften bevorzugten Ausführungsform der vorliegenden Erfindung. Wie bei den zuvor beschriebenen Ausführungsformen ist bei auch dieser fünften bevorzugten Ausführungsform innerhalb des Auges 1 das Elektrodenarray 6, der Tack 9, der Infrarot-Sender/Empfänger 8, 10 und die Drahtverbindung 5 zwischen dem intraokularen Teil und den extraokularen Komponenten der Sehprothese angeordnet. Die Sender/Empfänger-Spule 4 ist intrakorporal, aber außerhalb des Auges 1 angeordnet und kann sowohl elektromagnetische Wellen 2 senden als auch empfangen.

Rechts vom Auge 1 ist eine Sender/Empfänger-Spule 18 angeordnet, die sich außerhalb des Körpers befindet und über elektromagnetische Wellen 2 Signale an die Sender/Empfänger-Spule 4 induktiv übermittelt, die von der extraokularen über die Drahtverbindung 5 an den intraokularen Teil der Sehprothese weitergeleitet werden. Ähnlich der in Figur 5 dargestellten Ausführungsformen ist auch bei dieser fünften Ausführungsform keine separate Empfänger-Spule vorgesehen, sondern die extrakorporale Sender/Empfänger-Spule 18 kann ebenso wie die intrakorporale Sender/Empfänger-Spule 4, elektromagnetische Wellen 2 sowohl senden als auch empfangen. Über diese bidirektionale Schnittstelle zwischen der Sender/Empfänger-Spule 4 und der Sender/Empfänger-Spule 18 können Signale oder Daten induktiv von außerhalb des Auges 1 an den intraokularen Teil der Sehprothese übermittelt werden sowie umgekehrt Signale oder Daten induktiv von innerhalb des Auges 1 an den extrakorporalen Teil der Sehprothese in alterierender Betriebsweise übertragen werden, wie oben beschrieben.

Auch bei der in Figur 6 dargestellten fünften bevorzugten Ausführungsform ist ein Infrarot-Sender/Empfänger 8, 10 innerhalb des Auges 1 vorgesehen, der Daten vom intraokularen Teil der Sehprothese durch Infrarot-Signale 20 übermittelt, die über den natürlichen Lichtweg des Auges nach außen abstrahlen und von einem außerhalb des Körpers angeordneten Infrarot-Empfänger 8 aufgenommen werden. Ferner hat der extrakorporale Infrarot-Sender/Empfänger 8, 10 auch die Funktion eines Infrarot-Senders, der Daten durch Infrarot-Signale 20 übermittelt, die von außerhalb des Körpers über den natürlichen Lichtweg des Auges in das Auge gelangen und vom Infrarot-Sender/Empfänger 8, 10 des intraokularen Teils der Sehprothese aufgenommen werden. Auf diese Weise können Signale oder Daten mittels Infrarot-Signale 20 von außerhalb des Auges 1 an den intraokularen Teil der Sehprothese übermittelt werden sowie umgekehrt Signale oder Daten mittels Infrarot-Signale 20 von innerhalb des Auges 1 an den extrakorporalen Teil der Sehprothese übertragen werden.

Abweichend von den in den Figuren 3, 4 und 5 dargestellten Ausführungsformen sind bei dieser in Figur 6 gezeigten fünften Ausführungsform der Infrarot-Sender/Empfänger 8, 10 und die extrakorporale Sender/Empfänger-Spule 18 nicht miteinander in einer Vorrichtung kombiniert, sondern voneinander getrennt angeordnet. Der Infrarot-Sender/Empfänger 8, 10 umfasst wiederum eine parabolförmige Fläche mit lichtempfindlichen Sensoren, um die vom intraokularen Infrarot-Sender 10 über den natürlichen Lichtweg des Auges 1 nach außen gelangenden Infrarot-Signale 20 zuverlässig erfassen zu können.

### Liste der Bezugszeichen

- 1: menschliches Auge bzw. Augapfel
- 2: elektromagnetische Hochfrequenz-Signale
- 3: Retina-Stimulator-Chip bzw. RS-Chip
- 4: Hochfrequenz- Sender/Empfänger-Spule
- 5: Drahtverbindung zwischen RS-Chip 3 und Elektrodenarray 6
- 6: Elektrodenarray bzw. Mikrokontaktstruktur
- 7: Makula bzw. Stelle des schärfsten Sehens
- 8: Infrarot-Empfänger
- 9: Nagel bzw. Tack
- 10: Infrarot-Sender
- 11: Lederhaut bzw. Sklera
- 12: Aderhaut
- 13: Netzhaut bzw. Retina
- 14: Augenlinse
- 15: Ziliarmuskel
- 16: Bindehaut
- 17: Regenbogenhaut
- 18: Hochfrequenz-Sender/Empfänger-Spule
- 19: lichtemittierendes Element bzw. Infrarot-Leuchtdiode
- 20: Infrarot-Signale
- 21: Hornhaut
- 22: Gegenelektrode
- 23: Gegenelektrode
- 24: Gegenelektrode
- 25: Abzweigung der Drahtverbindung 5
- 26: Brille bzw. Brillengestell
- 27: Brillenbügel
- 28: Brillenglasrahmen
- S: Schnittebene

## Patentansprüche

1. Sehprothese mit einem Stimulations-System zur Implantation in einem menschlichen Auge mit einem Elektrodenarray (6) zur Kontaktierung und Stimulation von lebendem Gewebe oder Nerven im visuellen System des Auges (1), das mittels einer elektrischen Steuereinheit (3) Stimulations-Impulse erzeugt,
wobei das Stimulations-System zumindest ein intraokulares Implantat (6, 8) und zumindest ein extraokulares Implantat (3, 4) umfasst, welches das intraokulare Implantat (6, 8) mit Energie versorgt,
wobei das intraokulare Implantat (6, 8) mindestens einen Licht-Empfänger (8) umfasst, der ausgebildet ist, Licht-Signale (20) eines Licht-Senders (10) von außerhalb des Auges (1), vorzugsweise über den natürlichen Lichtweg des Auges (1) zu empfangen, **dadurch gekennzeichnet,**
**dass** der Licht-Empfänger des intraokularen Implantats (6, 8) als Infrarot-Empfänger ausgebildet ist, Infrarot-Signale (20) eines Infrarot-Senders (10) von außerhalb des Auges (1), vorzugsweise über den natürlichen Lichtweg des Auges (1) zu empfangen.

2. Sehprothese nach Anspruch 1, wobei das intraokulare Implantat (6, 8) mindestens ein lichtemittierendes Element (19) umfasst, das Licht-Signale in Abhängigkeit von Betriebsparametern des Stimulations-Systems ausstrahlt und das lichtemittierende Element (19) so im Augeninneren angeordnet ist, dass die von dem lichtemittierenden Element (19) abgestrahlten Licht-Signale von einem Beobachter über Sichtkontakt detektierbar sind.

3. Sehprothese nach Anspruch 1 ferner umfassend eine bidirektionale induktive Schnittstelle (4, 18) zur bidirektionalen Datenübertragung mit vorzugsweise mindestens zwei separaten Übertragungskanälen zwischen einem extrakorporalen Teil der Sehprothese und einem intrakorporalen Teil (3, 4, 6, 8), der das intraokulare Implantat (6, 8) und das extraokulare Implantat (3, 4) umfasst, und insbesondere wobei der extrakorporale Teil mindestens eine Antenne (18) außerhalb des Auges (1) für die bidirektionale induktive Schnittstelle (4, 18) umfasst, die elektromagnetische Signale (2) vorzugsweise im Hochfrequenz-Bereich senden und empfangen kann.

4. Sehprothese nach einem der vorangehenden Ansprüche, wobei der extrakorporale Teil mindestens zwei Antennen (18) für die bidirektionale induktive Schnittstelle (4, 18) umfasst, von denen eine erste Antenne elektromagnetische Signale (2) senden kann und eine zweite Antenne elektromagnetische Signale (2) empfangen kann.

5. Sehprothese nach einem der vorangehenden Ansprüche, wobei das intraokulare Implantat (6, 8) als epiretinales Implantat ausgebildet ist, das für eine Implantation innerhalb des Augapfels (1) auf der Netzhaut des Auges vorzugsweise im Bereich der Makula geeignet ist.

6. Sehprothese nach einem der vorangehenden Ansprüche, wobei das intraokulare Implantat (6, 8) ein Elektrodenarray (6) umfasst, in dem die Stimulations-Elektroden vorzugsweise in einer Matrix angeordnet sind, wobei das Elektrodenarray (6) des intraokularen Implantats (6, 8) eine Anzahl von Kontaktstellen zum Kontaktieren von Retinazellen bzw. Ganglienzellen aufweist, über die kontaktierte Retinazellen bzw. Ganglienzellen mittels Stimulations-Impulsen stimulierbar sind, insbesondere wobei das extraokulare Implantat (3, 4) die elektrische Steuereinheit (3) umfasst, welche die StimulationsImpulse erzeugt und vorzugsweise als digitale Steuereinheit mit analogen Zusatzfunktionen ausgebildet ist.

7. Sehprothese nach einem der vorangehenden Ansprüche, wobei die elektrische Steuereinheit (3) elektronische Speichermittel umfasst, um darin die Dauer und die Intensität der zu erzeugenden Stimulations-Impulse zu speichern, insbesondere wobei die elektronischen Komponenten der elektrischen Steuereinheit (3) zumindest teilweise in einem integrierten Schaltkreis z.B. photolithographisch mikrostrukturiert und vorzugsweise auf einem Chip in dem extraokularen Implantat untergebracht sind.

8. Sehprothese nach einem der vorangehenden Ansprüche, wobei die elektrische Steuereinheit für jede Stimulations-Elektrode ein Kontaktpad umfasst, um jeweils eine Stimulations-Elektrode über eine separate Drahtverbindung (5) zu kontaktieren, wobei das extraokulare Implantat (3, 4) mit dem intraokularen Implantat (6, 8) über eine Drahtverbindung (5) koppelbar ist, die mindestens eine Leitung zur Übertragung des Betriebsstroms und mindestens eine Signalleitung zur Übertragung elektrischer Stimulations-Impulse von der digitalen Steuereinheit (3) an das intraokulare Implantat (6, 8) umfasst.

9. Sehprothese nach Anspruch 7, wobei die Drahtverbindung (5) zwischen dem extraokularen Implantat (3, 4) und dem intraokularen Implantat (6, 8) mindestens so viele Leitungen zur Übertragung elektrischer Stimulations-Impulse umfasst, wie StimulationsElektroden in dem intraokularen Implantat (6, 8) vorgesehen sind.

10. Sehprothese nach einem der Ansprüche 8 bis 9, wobei die Drahtverbindung (5) ferner einen oder mehrere Lichtleiter insbesondere zur bidirektionalen Datenübertragung mittels Lichtsignalen zwischen dem extraokularen Implantat (3, 4) und dem intraokularen Implantat (6, 8) umfasst.

11. Sehprothese nach einem der Ansprüche 8 bis 10, die Drahtverbindung (5) zwischen dem extraokularen Implantat (3, 4) und dem intraokularen Implantat (6, 8) als flexibles Implantat ausgebildet ist, das vorzugsweise im Bereich der Parsplana von außerhalb des Augapfels (1) in das Augeninnere geführt ist.

12. Sehprothese nach einem der vorangehenden Ansprüche, wobei das intraokulare Implantat (6, 8) eine Anzahl von lichtempfindlichen Elementen umfasst, die in Abhängigkeit von auf dem intraokularen Implantat (6, 8) auftreffenden Licht die Kontaktstellen des Elektrodenarrays (6) über die elektrische Schaltung (3) ansteuern.

13. Sehprothese nach Anspruch 1, wobei die Schnittstelle (4, 18) zwischen dem Licht-Sender (10) außerhalb des Auges (1) und den lichtempfindlichen Elementen bzw. dem Licht-Empfänger (8) des intraokularen Implantats (6, 8) ausgebildet ist, von einer externen Kamera aufgenommene Bild-Daten über Licht-Signale (20) vom Licht-Sender (10) außerhalb des Auges (1) an die lichtempfindlichen Elemente bzw. den Licht-Empfänger (8) des intraokularen Implantats (6, 8) zu übermitteln.

14. Sehprothese nach Anspruch 1, wobei der Licht-Empfänger (8) und die elektrische Schaltung (3) über eine Drahtverbindung koppelbar sind, die zur Übertragung von BildDaten, vorzugsweise in Form eines seriellen Datenstroms dient, und/oder wobei der Licht-Empfänger (8) auf der Drahtverbindung (5), vorzugsweise im Bereich eines zur Fixierung der Drahtverbindung (5) dienenden Nagels (9) oder auf einer Abzweigung (25) von der Drahtverbindung (5) positioniert ist.

15. Sehprothese nach Anspruch 1, wobei das lichtemittierende Element (19) auf der Drahtverbindung (5), vorzugsweise im Bereich eines zur Fixierung der Drahtverbindung (5) dienenden Nagels (9) oder auf einer Abzweigung (25) von der Drahtverbindung (5) positioniert ist, wobei die von dem lichtemittierenden Element (19) abgestrahlten LichtSignale in Abhängigkeit von Betriebsparametern des intraokularen Implantats (6, 8) beispielsweise durch Modulation der Dauer und/oder der Intensität der Licht-Signale moduliert sind.

16. Sehprothese nach Anspruch 15, wobei die von dem lichtemittierenden Element (19) abgestrahlten Licht-Signale Informationen enthalten über die Position des intraokularen Implantats (6, 8), die Qualität der von dem intraokularen Implantat empfangenen BildDaten, über die Qualität der Stromversorgung des intraokularen Implantats (6, 8) und/oder über die Impedanz der Stimulations-Elektroden, wobei das lichtemittierende Element (19) so im Augeninneren angeordnet ist, dass die von dem lichtemittierenden Element (19) abgestrahlten Licht-Signale von einem Beobachter über Sichtkontakt detektierbar sind.

17. Sehprothese nach einem der vorangegangenen Ansprüche, wobei elektronische Komponenten, die zur Aufbereitung der von der externen Kamera erfassten Bild-Daten erforderlich sind und vorzugsweise auch die externe Kamera zusammen mit dem externen Infrarot-Sender bzw. mit dem externen Infrarot-Empfänger (10) in einem Brillengestell (26, 27, 28) untergebracht sind und/oder wobei die Antenne (18) des extrakorporalen Teils in einem Brillenglasrahmen (28) untergebracht ist.

18. Sehprothese nach einem der vorangegangenen Ansprüche, wobei der Infrarot-Empfänger (8) des extrakorporalen Teils eine parabolförmige lichtempfindliche Fläche aufweist, um die vom intraokularen Infrarot-Sender (10) abgestrahlten Signale (2) zu empfangen.

## Claims

1. Visual prosthesis with a stimulation system for implantation in a human eye, having an electrode array (6) for contacting and stimulating living tissue or nerves in the visual system of the eye (1), which generates stimulation impulses by means of an electrical control unit (3),
wherein the stimulation system comprises at least one intraocular implant (6, 8) and at least one extraocular implant (3, 4), which supplies the intraocular implant (6, 8) with energy,
wherein the intraocular implant (6, 8) comprises at least one light receiver (8), which is designed to receive light signals (20) of a light transmitter (10) from outside the eye (1), preferably via the natural light path of the eye (1), **characterized in that**
the light receiver of the intraocular implant (6, 8) is designed as an infrared receiver, to receive infrared signals (20) of an infrared transmitter (10) from outside the eye (1), preferably via the natural light path of the eye (1).

2. Visual prosthesis according to claim 1, wherein the intraocular implant (6, 8) comprises at least one light-emitting element (19), which radiates light signals as a function of operating parameters of the stimulation system and the light-emitting element (19) is arranged inside the eye so that the light signals emitted by the light-emitting element (19) can be detected by an observer via visual contact.

3. Visual prosthesis according to claim 1, furthermore comprising a bidirectional inductive interface (4, 18) for bidirectional data transmission, preferably with at least two separate transmission channels, between an extracorporeal part of the visual prosthesis and an intracorporeal part (3, 4, 6, 8), which comprises the intraocular implant (6, 8) and the extraocular implant (3, 4), and particularly wherein the extracorporeal part comprises at least one antenna (18) outside the eye (1) for the bidirectional inductive interface (4, 18), which can transmit and receive electromagnetic signals (2) preferably in the radiofrequency range.

4. Visual prosthesis according to one of the preceding claims, wherein the extracorporeal part comprises at least two antennas (18) for the bidirectional inductive interface (4, 18), of which a first antenna can transmit electromagnetic signals (2) and a second antenna can receive electromagnetic signals (2).

5. Visual prosthesis according to one of the preceding claims, wherein the intraocular implant (6, 8) is designed as an epiretinal implant, which is suitable for implantation inside the eyeball (1) on the retina of the eye, preferably in the region of the macula.

6. Visual prosthesis according to one of the preceding claims, wherein the intraocular implant (6, 8) comprises an electrode array (6), in which the stimulation electrodes are arranged preferably in a matrix, wherein the electrode array (6) of the intraocular implant (6, 8) has a number of contact sites for contacting retinal cells or ganglion cells, via which contacted retinal cells or ganglion cells can be stimulated by means of stimulation impulses, particularly wherein the extraocular implant (3, 4) comprises the electrical control unit (3), which generates the stimulation impulses and is preferably designed as a digital control unit with analogue auxiliary functions.

7. Visual prosthesis according to one of the preceding claims, wherein the electrical control unit (3) comprises electronic storage means, in order to store therein the duration and the intensity of the stimulation impulses to be generated, particularlywherein the electronic components of the electrical control unit (3) are accommodated at least partially in an integrated circuit, for example by being photolithographically microstructured, and preferably on a chip in the extraocular implant.

8. Visual prosthesis according to one of the preceding claims, wherein the electrical control unit comprises a contact pad for each stimulation electrode, for respectively contacting a stimulation electrode via a separate wire connection (5), wherein the extraocular implant (3, 4) can be coupled to the intraocular implant (6, 8) via a wire connection (5), which comprises at least one line for transmitting the operating current and at least one signal line for transmitting electrical stimulation impulses from the digital control unit (3) to the intraocular implant (6, 8).

9. Visual prosthesis according to claim 7, wherein the wire connection (5) between the extraocular implant (3, 4) and the intraocular implant (6, 8) comprises at least as many lines for transmitting electrical stimulation impulses as there are stimulation electrodes in the intraocular implant (6, 8).

10. Visual prosthesis according to one of claims 8 to 9, wherein the wire connection (5) furthermore comprises one or more optical fibers, in particular for bidirectional data transmission by means of light signals between the extraocular implant (3, 4) and the intraocular implant (6, 8).

11. Visual prosthesis according to one of claims 8 to 10, wherein the wire connection (5) between the extraocular implant (3, 4) and the intraocular implant (6, 8) is designed as a flexible implant, which is preferably fed from outside the eyeball (1) into the interior of the eye in the region of the pars plana.

12. Visual prosthesis according to one of the preceding claims, wherein the intraocular implant (6, 8) comprises a number of photosensitive elements, which drive the contact sites of the electrode array (6) via the electrical circuit (3) as a function of light incident on the intraocular implant (6, 8).

13. Visual prosthesis according to claim 1, wherein the interface (4, 18) between the light transmitter (10) outside the eye (1) and the photosensitive elements or the light receiver (8) of the intraocular implant (6, 8) is designed to transfer image data captured by an external camera via light signals (20) from the light transmitter (10) outside the eye (1) to the photosensitive elements or the light receiver (8) of the intraocular implant (6, 8).

14. Visual prosthesis according to claim 1, wherein the light receiver (8) and the electrical circuit (3) can be coupled via a wire connection which is used to transmit image data, preferably in the form of a serial data stream, and/orwherein the light receiver (8) is positioned on the wire connection (5), preferably in the region of a nail or tack (9) used for fixing the wire connection (5) or on a branch (25) of the wire connection (5).

15. Visual prosthesis according to claim 1, wherein the light-emitting element (19) is positioned on the wire connection (5), preferably in the region of a nail or tack (9) used for fixing the wire connection (5) or on a branch (25) of the wire connection (5), wherein the light signals emitted by the light-emitting element (19) are modulated as a function of operating parameters of the intraocular implant (6, 8), for example by modulating the duration and/or the intensity of the light signals.

16. Visual prosthesis according to claim 15, wherein the light signals emitted by the light-emitting element (19) contain information about the position of the intraocular implant (6, 8), the quality of the image data received by the intraocular implant, about the quality of the current supply of the intraocular implant (6, 8) and/or about the impedance of the stimulation electrodes, wherein the light-emitting element (19) is arranged inside the eye so that the light signals emitted by the light-emitting element (19) can be detected by an observer via visual contact.

17. Visual prosthesis according to one of the preceding claims, wherein electronic components which are required for preparing the image data captured by the external camera, and preferably also the external camera, are accommodated together with the external infrared transmitter or with the external infrared receiver (10) in a spectacle frame (26, 27, 28), and/or wherein the antenna (18) of the extracorporeal part is accommodated in a spectacle frame (28).

18. Visual prosthesis according to one of the preceding claims, wherein the infrared receiver (8) of the extracorporeal part has a parabolic photosensitive surface, in order to receive signals (2) emitted by the intraocular infrared transmitter (10).

## Revendications

1. Prothèse visuelle avec un système de stimulation destiné à être implanté dans un oeil humain, avec une matrice d'électrodes (6) pour le contact et la stimulation de tissus vivants ou de nerfs dans le système visuel de l'oeil (1), qui génère, à l'aide d'une unité de commande électrique (3), des impulsions de stimulation,
le système de stimulation comprenant au moins un implant intraoculaire (6, 8) et au moins un implant extraoculaire (3, 4) qui alimente en énergie l'implant intraoculaire (6, 8),
l'implant intraoculaire (6, 8) comprenant au moins un récepteur de lumière (8) conçu pour recevoir des signaux lumineux (20) d'un émetteur de lumière (10) de l'extérieur de l'oeil (1), de préférence sur le chemin optique naturel de l'oeil (1),
**caractérisée en ce que** le récepteur de lumière (8) de l'implant intraoculaire (6, 8) est conçu comme un récepteur infrarouge afin de recevoir des signaux infrarouges (20) d'un émetteur infrarouge (10) de l'extérieur de l'oeil (1), de préférence sur le chemin optique naturel de l'oeil (1).

2. Prothèse visuelle selon la revendication 1, dans laquelle l'implant intraoculaire (6, 8) comprend au moins un élément émetteur de lumière (19) qui émet des signaux lumineux en fonction de paramètres de fonctionnement du système de stimulation, et l'élément émetteur de lumière (19) est disposé à l'intérieur de l'oeil de façon à ce que les signaux lumineux émis par l'élément émetteur de lumière (19) puissent être détectés par un observateur à l'aide d'un contact visuel.

3. Prothèse visuelle selon la revendication 1, comprenant en outre une interface inductive bidirectionnelle (4, 18) permettant la transmission bidirectionnelle de données avec de préférence au moins deux canaux de transmission séparés entre une partie extracorporelle de la prothèse visuelle et une partie intracorporelle (3, 4, 6, 8), qui comprend l'implant intraoculaire (6, 8) et l'implant extraoculaire (3, 4), et en particulier dans laquelle la partie extracorporelle comprend au moins une antenne (18) à l'extérieur de l'oeil (1) pour l'interface inductive bidirectionnelle (4, 18) qui peut émettre et recevoir des signaux électromagnétiques (2), de préférence dans le domaine des hautes fréquences.

4. Prothèse visuelle selon l'une quelconque des revendications précédentes, dans laquelle la partie extracorporelle comprend au moins deux antennes (18) pour l'interface inductive bidirectionnelle (4, 18), dont une première antenne peut émettre des signaux électromagnétiques (2) et une deuxième antenne peut recevoir des signaux électromagnétiques (2).

5. Prothèse visuelle selon l'une quelconque des revendications précédentes, dans laquelle l'implant intraoculaire (6, 8) est conçu comme un implant épirétinal destiné à être implanté à l'intérieur du globe oculaire (1) sur la rétine de l'oeil, de préférence au niveau de la macula.

6. Prothèse visuelle selon l'une quelconque des revendications précédentes, dans laquelle l'implant intraoculaire (6, 8) comprend une matrice d'électrodes (6) dans laquelle des électrodes de stimulation sont disposées de préférence sous la forme d'une matrice, dans laquelle la matrice d'électrodes (6) de l'implant intraoculaire (6, 8) présente plusieurs points de contact permettant le contact avec des cellules rétiniennes ou des cellules ganglionnaires, grâce auxquels les cellules rétiniennes ou les cellules ganglionnaires en contact peuvent être stimulées à l'aide d'impulsions de stimulation, en particulier dans laquelle l'implant extraoculaire (3, 4) comprend l'unité de commande électrique (3) qui génère les impulsions de stimulation et est conçue de préférence comme une unité de commande numérique avec des fonctions supplémentaires analogiques.

7. Prothèse visuelle selon l'une quelconque des revendications précédentes, dans laquelle l'unité de commande électrique (3) comprend des moyens de stockage électroniques afin d'y stocker la durée et l'intensité des impulsions de stimulation générées, en particulier dans laquelle les composants électroniques de l'unité de commande électrique (3) sont par exemple micro-structurés par photolithographie au moins partiellement dans un circuit intégré et de préférence logés sur une puce dans l'implant extraoculaire.

8. Prothèse visuelle selon l'une quelconque des revendications précédentes, dans laquelle l'unité de commande électrique comprend pour chaque électrode de stimulation une plage de contact afin de mettre en contact respectivement une électrode de stimulation par l'intermédiaire d'une liaison filaire (5) séparée, dans laquelle l'implant extraoculaire (3, 4) peut être couplé à l'implant intraoculaire (6, 8) par l'intermédiaire d'une liaison filaire (5) qui comprend au moins une ligne pour la transmission du courant de fonctionnement et au moins une ligne de signal pour la transmission d'impulsions de stimulation électriques de l'unité de commande numérique (3) à l'implant intraoculaire (6, 8).

9. Prothèse visuelle selon la revendication 7, dans laquelle la liaison filaire (5) entre l'implant extraoculaire (3, 4) et l'implant intraoculaire (6, 8) comprend au moins autant de lignes de transmission des impulsions électriques que d'électrodes de stimulation prévues dans l'implant intraoculaire (6, 8).

10. Prothèse visuelle selon l'une quelconque des revendications 8 à 9, dans laquelle la liaison filaire (5) comprend en outre une ou plusieurs fibres optiques, en particulier pour la transmission de données bidirectionnelle à l'aide de signaux lumineux entre l'implant extraoculaire (3, 4) et l'implant intraoculaire (6, 8).

11. Prothèse visuelle selon l'une quelconque des revendications 8 à 10, dans laquelle la liaison filaire (5) entre l'implant extraoculaire (3, 4) et l'implant intraoculaire (6, 8) est conçue comme un implant flexible qui est guidé de préférence au niveau de la pars plana de l'extérieur du globe oculaire (1) vers l'intérieur de l'oeil.

12. Prothèse visuelle selon l'une quelconque des revendications précédentes, dans laquelle l'implant intraoculaire (6, 8) comprend plusieurs éléments photosensibles qui commandent, en fonction de la lumière incidente sur l'implant intraoculaire (6, 8), les points de contact de la matrice d'électrodes (6) par l'intermédiaire du circuit électrique (3).

13. Prothèse visuelle selon la revendication 1, dans laquelle l'interface (4, 18) entre l'émetteur de lumière (10) à l'extérieur de l'oeil (1) et les éléments photosensibles ou le récepteur de lumière (8) de l'implant intraoculaire (6, 8) est conçue pour transmettre des données d'images enregistrées par une caméra externe à l'aide de signaux lumineux (20) provenant de l'émetteur de lumière (10) à l'extérieur de l'oeil (1) aux éléments photosensibles ou au récepteur de lumière (8) de l'implant intraoculaire (6, 8).

14. Prothèse visuelle selon la revendication 1, dans laquelle le récepteur de lumière (8) et le circuit électrique (3) peuvent être couplés par l'intermédiaire d'une liaison filaire servant à transmettre des données d'images, de préférence sous la forme d'un flux de données séquentiel, et/ou dans laquelle le récepteur de lumière (8) est positionné sur la liaison filaire (5), de préférence au niveau d'un goujon (9) servant à fixer la liaison filaire (5) ou sur un embranchement (25) de la liaison filaire (5).

15. Prothèse visuelle selon la revendication 1, dans laquelle l'élément émetteur de lumière (19) est positionné sur la liaison filaire (5), de préférence au niveau d'un goujon (9) servant à fixer la liaison filaire (5) ou sur un embranchement (25) de la liaison filaire (5), dans laquelle les signaux lumineux émis par l'élément émetteur de lumière (19) sont modulés en fonction de paramètres de fonctionnement de l'implant intraoculaire (6, 8), par exemple par une modulation de la durée et/ou de l'intensité des signaux lumineux.

16. Prothèse visuelle selon la revendication 15, dans laquelle les signaux lumineux émis par l'élément émetteur de lumière (19) contiennent des informations concernant la position de l'implant intraoculaire (6, 8), la qualité des données d'images reçues par l'implant intraoculaire, la qualité de l'alimentation électrique de l'implant intraoculaire (6, 8) et/ou l'impédance des électrodes de stimulation, dans laquelle l'élément émetteur de lumière (19) est disposé à l'intérieur de l'oeil de façon à ce que les signaux lumineux émis par l'élément émetteur de lumière (19) puissent être détectés par un observateur à l'aide d'un contact visuel.

17. Prothèse visuelle selon l'une quelconque des revendications précédentes, dans laquelle des composants électroniques, nécessaires pour le traitement des données d'images enregistrées par une caméra externe, et de préférence également la caméra externe, sont logés ensemble avec l'émetteur infrarouge externe (10) dans une monture de lunettes (26, 27, 28), et/ou dans laquelle l'antenne (10) de la partie extracorporelle est logée dans une monture de lunettes (28).

18. Prothèse visuelle selon l'une quelconque des revendications précédentes, dans laquelle le récepteur infrarouge (8) de la partie extracorporelle présente une surface photosensible de forme parabolique permettant de recevoir les signaux (2) émis par l'émetteur infrarouge intraoculaire (10).
